# EUROPEAN PATENT SPECIFICATION

(11) **EP 2 999 790 B1**
(45) Date of publication and mention of the grant of the patent: **31.10.2018**
(21) Application number: 14727372.6
(22) Date of filing: 23.05.2014
(51) Int. Cl.: C12N 15/82, C07K 14/415, C07K 14/32

(54) **PROCESS OF PROVIDING PLANTS WITH ABIOTIC STRESS RESISTANCE**
VERFAHREN ZUR BEREITSTELLUNG VON PFLANZEN MIT RESISTENZ GEGENÜBER ABIOTISCHEM STRESS
PROCÉDÉ DE PRODUCTION DE PLANTES À RÉSISTANCE AU STRESS ABIOTIQUE

(30) Priority: 23.05.2013 EP 13002691
(43) Date of publication of application: 30.03.2016
(73) Proprietor: Nomad Bioscience GmbH, 80333 München (DE)
(72) Inventor: THÜMMLER, Anka, 06114 Halle (Saale) (DE); BARTELS, Doreen, 06366 Köthen (Anhalt) (DE); GIRITCH, Anatoli, 06108 Halle (Saale) (DE); GLEBA, Yuri, 10117 Berlin (DE)
(74) Representative: Blodig, Wolfgang
(86) International application number: PCT/EP2014/001403
(87) International publication number: WO 2014/187571

(56) References cited:
- WO-A1-2008/043268
- WO-A1-2012/019660
- WO-A2-2005/033318
- WO-A2-2010/019838
- XIAO-YUN JIA1 ET AL: "Molecular cloning and characterization of wheat calreticulin (CRT) gene involved in drought-stressed responses", JOURNAL OF EXPERIMENTAL BOTANY, OXFORD UNIVERSITY PRESS, GB, vol. 59, no. 4, 1 January 2008 (2008-01-01), pages 739-751, XP007922149, ISSN: 0022-0957, DOI: 10.1093/JXB/ERM369
- MINGGUI YANG ET AL: "Overexpression of the Brassica napus BnLAS gene in Arabidopsis affects plant development and increases drought tolerance", PLANT CELL REPORTS, vol. 30, no. 3, 1 March 2011 (2011-03-01), pages 373-388, XP055135103, ISSN: 0721-7714, DOI: 10.1007/s00299-010-0940-7
- HONGMEI WANG ET AL: "Expression of an apoplast-localized BURP-domain protein from soybean (GmRD22) enhances tolerance towards abiotic stress", PLANT, CELL & ENVIRONMENT, vol. 35, no. 11, 22 November 2012 (2012-11-22), pages 1932-1947, XP055135104, ISSN: 0140-7791, DOI: 10.1111/j.1365-3040.2012.02526.x
- GIRITCH A ET AL: "Transient Expression Technologies as an Alternative to Genetically Modified Plants", INTERNET CITATION, 1 January 2011 (2011-01-01), page 1, XP002676667, Retrieved from the Internet: URL:http://conference.icbge.org.ua/en/abst racts/30-transient-expression-technologies -as-an-alternative-to-genetically-modified -plants [retrieved on 2012-05-23]
- YANG SHUJUN ET AL: "Narrowing Down the Targets: Towards Successful Genetic Engineering of Drought-Tolerant Crops", MOLECULAR PLANT, OXFORD UNIVERSITY PRESS, GB, vol. 3, no. 3, 1 May 2010 (2010-05-01), pages 469-490, XP009147655, ISSN: 1674-2052, DOI: 10.1093/MP/SSQ016
- ZVI PELEG ET AL: "Hormone balance and abiotic stress tolerance in crop plants", CURRENT OPINION IN PLANT BIOLOGY, vol. 14, no. 3, 4 March 2011 (2011-03-04), pages 290-295, XP028223979, ISSN: 1369-5266, DOI: 10.1016/J.PBI.2011.02.001 [retrieved on 2011-02-17]

## Description

### FIELD OF THE INVENTION

The present invention relates to a process of providing a plurality of plants with increased resistance against drought stress.

### BACKGROUND OF THE INVENTION

Abiotic stress tolerance of plants is of great importance for agriculture under harsh environmental conditions, since seed, fruit and biomass production of plants can be impaired substantially by drought or other abiotic stresses that may occur during the vegetation period. Attempts have already been undertaken to increase the tolerance of crop plants to various abiotic stresses (e.g. cold stress, heat stress, water stress, salt stress, drought) by biotechnological means. WO 2005/033318 relates to methods for enhancing stress tolerance in plants, and describes transgenic plants stably transformed with a DNA encoding a cold shock protein from gram positive bacteria, such as *Bacillus subtilis.* Increased abiotic stress tolerance is reported for transgenic plants. One cold shock protein mentioned in WO 2005/033318 is *B. subtilis* CspB.

Castiglioni et al., Plant Physiology 147 (2008) 446-455 relates to bacterial RNA chaperones that confer abiotic stress tolerance in plants and improved grain yield in maize under water-limited conditions. Stress tolerance at both vegetative and reproductive stages is reported with enhanced yield stability in maize under water-limiting conditions. Also in this study, plants transgenic for genes encoding cold shock proteins were used.
Jia et al., Journal of Experimental Botany 59(4) (2008) 739-751 reports research on the role of the calreticulin (CRT) gene in plant response against drought.
WO 2010/019838 A2 relates to the production of transgenic plants with enhanced stress tolerance and/or enhanced yield.
WO 2012/019660 A1 describes a process of transfecting a plant, comprising spraying parts of said plant with an aqueous suspension containing cells of an *Agrobacterium* strain and at least one abrasive suspended in said suspension.

Abiotic stress is due to environmental conditions and thus generally affects the entire plant. This seems to be the reason as to why transgenic plants have been used in the prior art for providing plants with proteins that increase stress tolerance. However, generation of transgenic crop plant lines is a time-consuming process, requiring many years of development. Moreover, planting of transgenic crops is not allowed or severely restricted (such as to use as animal feed) in several countries, whereby the approaches described in the prior art cannot be used in such countries. Further, transgenic plants generally contain selectable marker genes that were used for selecting transformed plants or cells thereof. Use of such selectable marker genes in transgenic plants is a major concern by critics of transgenic plants, as spread of antibiotic resistance genes may impair the utility of antibiotics in medicine, and spread of herbicide resistance genes to weeds may impair the utility of herbicides. Thus, the abiotic stress protection methods of WO 2005/033318 and Castiglioni et al. are ecologically problematic.

It is an object of the invention to provide a process of providing a plant with increased abiotic stress (notably drought) resistance. It is another object of the invention to provide a process of providing a plant with increased abiotic stress (notably drought) resistance, which is of improved environmental safety and/or does not require spread of antibiotic or herbicide resistance genes in the environment. It is another object of the invention to provide a process of providing crop plants with increased abiotic stress (notably drought) resistance that can be used in countries where planting of transgenic crop plants is not allowed.

### SUMMARY OF THE INVENTION

Accordingly, the present invention provides the following:
(1) A process of providing a plurality of plants with improved drought stress tolerance, wherein the plants are monocot or dicot plants, comprising
   growing said plurality of plants on a farm field;
   determining whether drought stress affects said plants;
   providing aerial parts of said plants with a suspension containing cells of an *Agrobacterium* strain comprising a nucleic acid molecule comprising a nucleic acid construct containing a nucleotide sequence of interest, said nucleotide sequence of interest providing said plants with increased drought stress resistance upon expression in said plants, wherein said plants are transiently transfected with said nucleic acid molecule.
(2) The process according to item 1 , wherein, before said step of providing aerial parts of said plant with said suspension, said plant or said plurality of plants does not contain a gene that provides said plant(s) with said abiotic stress tolerance.
(3) The process according to item 1 or 2, wherein said nucleotide sequence of interest encodes a protein capable of providing said plant(s) with said increased drought stress tolerance.
(4) The process according to item 3, wherein said protein
   - comprises or consists of an amino acid sequence of any one of the amino acid sequences of SEQ ID NO: 2, 4, 6, 8, 10, 12, 14, 16, 18, 20, 22, and 24; or
   - comprises or consists of an amino acid sequence having at least 80% sequence identity to the entire amino acid sequence of any one of SEQ ID NO: 2, 4, 6, 8, 10, 12, 14, 16, 18, 20, 22, and 24; or
   - comprises or consists of an amino acid sequence of a length of at least 80 %, preferably at least 90 %, more preferably at least 95% of the number of amino acid residues of any one of SEQ ID NO: 2, 4, 6, 8, 10, 12, 14, 16, 18, 20, 22, and 24 and a sequence identity of at least 90% to SEQ ID NO: 2, 4, 6, 8, 10, 12, 14, 16, 18, 20, 22, and 24, respectively, over such length; or
   - comprises or consists of an amino acid sequence having from 1 to 20 amino acid additions, substitutions or deletions compared to the amino acid sequence of any one of SEQ ID NO: 2, 4, 6, 8, 10, 12, 14, 16, 18, 20, 22, and 24.
(5) The process according to item 3, wherein said protein has a cold shock domain, preferably said protein is an RNA chaperone having a binding site for single-stranded RNA.
(6) The process according to any one of items 3 to 5, wherein said protein comprises the amino acid sequence of SEQ ID NO: 2 or of SEQ ID NO: 4,
   or said protein comprises an amino acid sequence having at least 80 % identity over the entire range of SEQ ID NO: 1 or NO:2.
(7) The process according to item 6, wherein said protein having at least 80 % identity over the entire range of SEQ ID NO:2 has at least the amino acid residues of CspA from E. coli W11, F18, F20, F31, H33, and F34 at positions corresponding to these positions in CspA from E. coli.
(8) The process according to any one of items 3 to 7, wherein said protein is CspB from B. subtilis.
(9) The process according to any one of items 1 to 8, wherein said suspension that may be aqueous contains said cells of said Agrobacterium strain in a concentration of at most 2.2·107, preferably of at most 1.1·107, more preferably of at most 4.4·106, more preferably of at most 1.1·106 cfu/ml of said suspension.
(10) The process according to any one of items 1 to 9, wherein said suspension further contains and abrasive suspended in said suspension, said abrasive preferably being a particulate inorganic carrier for wettable powders, such as silica or carborundum.
(11) The process according to item 10, wherein said suspension contains said abrasive in an amount of between 0.02 and 2, preferably between 0.05 and 1 and more preferably between 0.1 and 0.5 % by weight of said suspension.
(12) The process according to any one of items 1 to 11, wherein said suspension further comprises an agricultural spray adjuvant, preferably a non-ionic surfactant or wetting agent, preferably the spray adjuvant is an organo-silicone wetting agent, such as Silwet L-77.
(13) The process according to any one of items 1 to 12, wherein said nucleic acid construct is flanked by a T-DNA border sequence on at least one side, which permits the transfer of said nucleic acid construct into cells of said plant.
(14) The process according to any one of items 1 to 13, wherein said nucleic acid construct is present in T-DNA and flanked both sides by T-DNA border sequences, said T-DNA not containing a selectable marker allowing selection of plant or plant cell containing said T-DNA.
(15) The process according to any one of items 1 to 14, wherein said nucleotide sequence of interest is operably linked to a promoter active in plant cells.
(16) The process according to any one of items 1 to 15, wherein said nucleic acid construct encodes a DNA or RNA replicon encoding said protein.

The invention further describes the following:
(20) DNA molecule or vector for Agrobacterium-mediated transformation of plants, comprising in T-DNA a nucleic acid construct containing a DNA sequence of interest operably linked to a promoter; said DNA sequence of interest encoding a protein capable of providing a plant with increased abiotic stress tolerance; said T-DNA not containing a selectable marker allowing selection of plant cells containing said T-DNA.
(21) Agrobacterium cell comprising the DNA molecule of item 20.
(22) Agrobacterium strain comprising a heterologous DNA molecule comprising in T-DNA a nucleic acid construct containing a heterologous DNA sequence of interest operably linked to a promoter; said heterologous DNA sequence of interest encoding a protein capable of providing a plant with increased drought resistance; said T-DNA not containing a selectable marker allowing selection of plant cells containing said T-DNA.
(23) Composition containing cells of an Agrobacterium strain, said Agrobacterium strain comprising a heterologous DNA molecule comprising a nucleic acid construct containing a heterologous DNA sequence of interest operably linked to a promoter; said nucleotide sequence of interest providing a plant with increased abiotic stress resistance upon expression in said plant; said composition optionally further comprising a preferably non-ionic wetting agent such as an organosilicone surfactant.
(24) The composition according to item 23, further comprising at least one abrasive.
(25) The composition according to item 23 or 24, wherein said composition is a suspension containing cells of said Agrobacterium strain and at least one abrasive suspended in said suspension, wherein said suspension contains said cells of said Agrobacterium strain in a concentration of at most 4.4·107, preferably of at most 1.1·107, preferably of at most 4.4·106, more preferably of at most 1.1·106 cfu/ml of said suspension.
(27) A plant treated with a composition containing cells of an Agrobacterium strain comprising a nucleic acid molecule comprising a nucleic acid construct containing a nucleotide sequence of interest, said nucleotide sequence of interest providing said plant with increased abiotic stress resistance upon expression in said plant; said plant containing in cells thereof said nucleotide sequence of interest and expresses said nucleotide sequence of interest.
(28) Use of the DNA molecule of item 20, the Agrobacterium cell of item 21, the Agrobacterium strain of item 22 or the composition of any one of items 23 to 25 for conferring abiotic stress tolerance to a plant.
(29) Method of conferring abiotic stress tolerance to a plant, comprising the following steps: preparing a suspension containing cells of an *Agrobacterium* strain comprising a nucleic acid molecule comprising a nucleic acid construct containing a nucleotide sequence of interest, said nucleotide sequence of interest providing said plant with increased abiotic stress resistance upon expression in said plant; and applying the suspension prepared in the previous step to aerial parts of said plant.

The inventors have surprisingly found that it is possible to achieve increased abiotic stress resistance such as drought resistance in plants by transiently expressing in plants endangered by an abiotic stress a nucleotide sequence of interest providing said plant with increased abiotic stress resistance upon expression in said plant. In one embodiment, the nucleotide sequence of interest encodes a protein capable of providing said plants with increased abiotic stress resistance. Disclosed is an abiotic stress protection system, kit and method for plants, wherein provision of a foreign gene (such as the nucleotide sequence of interest) to said plant can be restricted to environmental conditions where abiotic stress such as drought occurs or is expected to occur. If no abiotic stress occurs or is to be expected, providing a foreign gene or transgene e.g. encoding said protein to the plants is not necessary and can be avoided. Consequently, the process of the invention is useful even for countries where agriculture employing transgenic plants (containing a foreign gene stably and heritably incorporated into a chromosome) is not allowed.

The invention enables a crop management system wherein plants that were provided with increased abiotic stress resistance according to the processes of the invention may be excluded from being used for food production for humans, but may instead be used for animal feed production or for biofuel production. However, plants that were not provided with increased abiotic stress resistance due to favourable environmental conditions in a growing season may be used for human food production. The invention represents a large improvement compared to prior art abiotic stress improvement methods (WO 2005/033318; Castiglioni et al., Plant Physiology 147 (2008) 446-455) wherein transgenic plants are planted even in growing seasons where no improved stress tolerance is needed.

Since the nucleotide sequence of interest that is, upon expression, capable of providing said plants with increased abiotic stress resistance is provided to said plants transiently, i.e. without the need for a selectable marker gene such as an antibiotic resistance gene or a herbicide resistance gene and without selecting for plant cells having incorporated such antibiotic resistance gene or a herbicide resistance gene, no antibiotic resistance gene or a herbicide resistance gene is inserted into plants, and spread of such genes in the environment with these plants can be largely avoided. Thus, also for this reason, the process of the invention has improved environmental safety compared to the abiotic stress protection systems of the prior art that rely on transgenic plants. Providing the nucleotide sequence of interest of the invention transiently to plants under abiotic stress conditions provides a selective advantage for the treated plants.

### BRIEF DESCRIPTION OF THE FIGURES

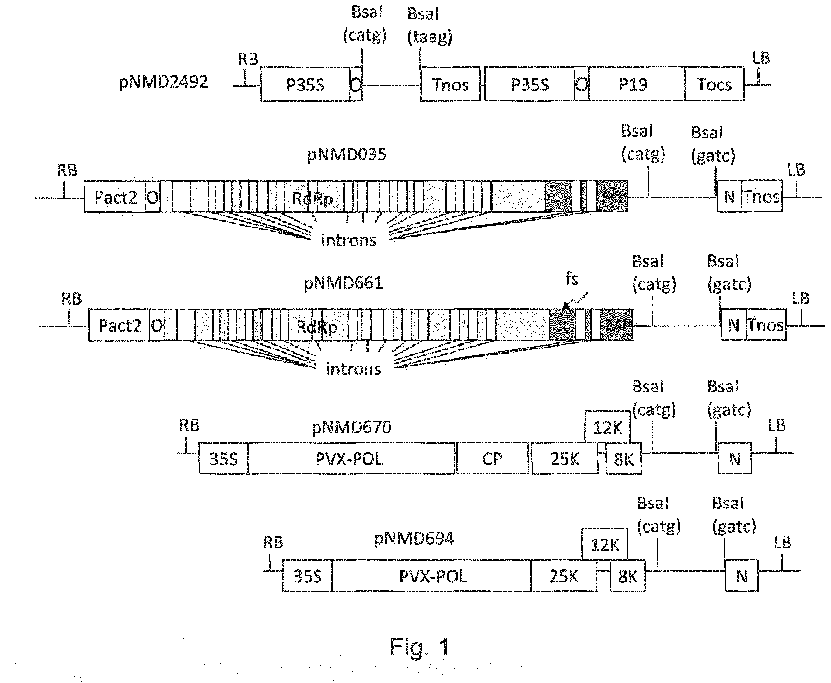

Fig. 1 shows schematically plasmid vectors used for cloning of genes of interest. **pNMD2492** is a transcriptional non-replicating vector. RB and LB stand for the right and left borders of T-DNA. P35S: cauliflower mosaic virus 35S promoter; O: omega translational enhancer; P19: PTGS suppressor P19 from Tomato Bushy Stunt Virus; Tnos: nopaline synthase terminator; Tocs: ocs terminator. **pNMD035** is a TMV-based viral vector with cell-to cell movement ability. Pact2: promoter of *Arabidopsis* actin2 gene; o: 5' end from TVCV (turnip vein clearing virus); RdRp: RNA-dependent RNA polymerase open reading frame (ORF) from cr-TMV (crucifer-infecting tobamovirus); MP: movement protein ORF from cr-TMV; N: 3'-non-translated region from cr-TMV; Tnos or nos: nopaline synthase terminator; white segments interrupting grey segments in the RdRp and MMP ORFs indicate introns inserted into these ORFs for increasing the likelihood of RNA replicon formation in the cytoplasm of plant cells, which is described in detail in WO2005049839. **pNMD661** is a TMV-based vector lacking cell-to cell movement ability. A point mutation in the MP ORF leads to a frame shift preventing correct MP translation. **pNMD670** is a PVX (potato virus X)-based vectors with cell-to-celi movement ability. PVX-pol: RNA-dependent RNA polymerase from PVX; CP: coat protein ORF: 25K, 12K and 8 together indicate the 25KDA, 12 kDa and 8 kDa triple gene block modules from PVX; N: 3'-untranslated region from PVX. **pNMD694** is a PVX-based vector with deletion of the coat protein coding sequence were disabled for both systemic and cell-to cell movement.
Fig. 2 shows schematically transcriptional vectors for the expression of *Arabidopsis thaliana* nuclear transcription factor Y subunit B-1 (NF-YB1) (GenBank: NM_129445.2) (plasmid construct pNMD3486) and Cold shock protein CspB from *Bacillus subtilis* subsp. *subtilis* str JH642 (GenBank: AAB01346) (plasmid construct pNMD3493).
Fig. 3 shows *Brassica napus* N90-740 plants 11 days post spraying, 11 days drought.
Fig. 4 shows *Brassica napus* N90-740 plants 18 days post spraying, 11 days drought, 7 days rewatering.
Fig. 5 shows *Brassica napus* N90-740 plants 26 days post spraying, 11 days drought, 7 days rewatering, 8 days drought.
Fig. 6 shows *Brassica napus* N90-740 plants 26 days post spraying, 11 days drought, 7 days rewatering, 13 days drought, 1 day rewatering.

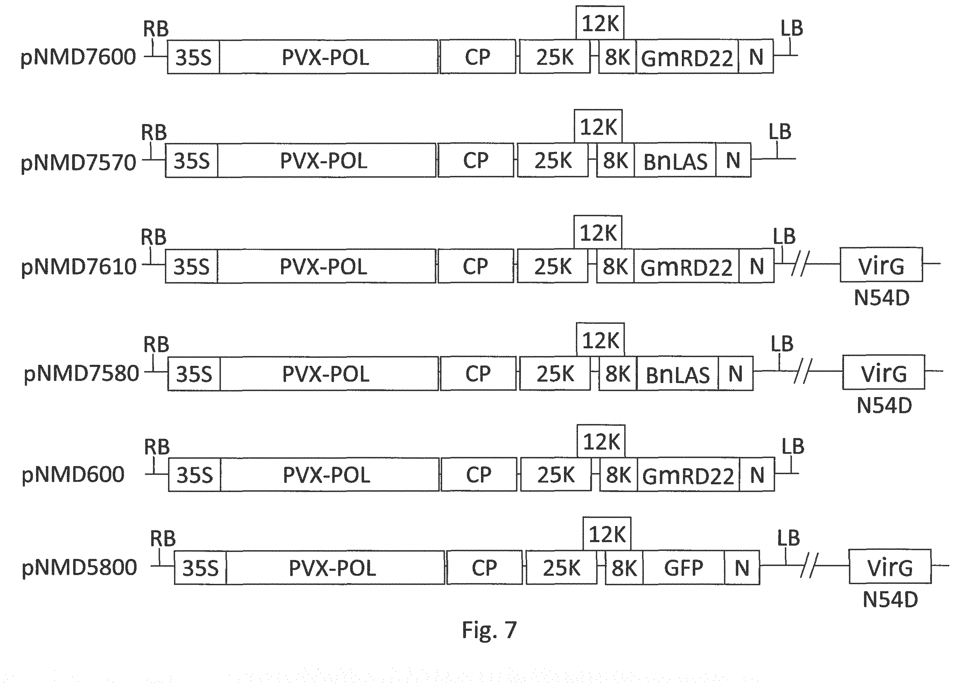

Fig. 7 shows schematically PVX viral vectors with systemic movement ability used for expression of genes of interest in tomato plants. RB and LB stand for the right and left borders of T-DNA. P35S: cauliflower mosaic virus 35S promoter; PVX-POL: RNA-dependent RNA polymerase from PVX; CP: coat protein ORF; 25K, 12K and 8 together indicate the 25KDA, 12 kDa and 8 kDa triple gene block modules from PVX; N: 3'-untranslated region from PVX; GmRD22: coding sequence of GmRD22 (apoplast-localized BURP-domain protein) from soybean *Glycine max;* BnLAS: coding sequence of BnLAS (GRAS family gene) from rapeseed *Brassica napus;* GFP: coding sequence of Green Fluorescent Protein from jellyfish. VirG N54D stands for the coding sequence of VirG protein from *Agrobacterium tumefaciens* GV3101 with N54D mutation.
Fig. 8 shows phenotypes of transfected tomato *Lycopersicon esculentum* cv. Balcony Red plants exposed to drought three weeks post spraying with agrobacteria harboring PVX vectors for the expression of BnLAS and GmRD22 genes. Drought exposure was performed for 14 days.
Fig. 9 shows fruits of transfected tomato *Lycopersicon esculentum* cv. Balcony Red plants exposed to drought three weeks post spraying with agrobacteria harboring PVX vectors for the expression of BnLAS and GmRD22 genes. Drought exposure was performed for 14 days. Fruit yield was evaluated 100 days post spraying. Numerals 1, 2, 3 etc. stand for individual plants.
Fig. 10 shows biomass of fruits harvested from transfected tomato *Lycopersicon esculentum* cv. Balcony Red plants exposed to drought three weeks post spraying with agrobacteria harboring PVX vectors. Drought exposure was performed for 14 days. Fruit yield was evaluated 100 days post spraying and calculated as an average per plant. 1: untransfected plant; 2: plants expressing BnLAS gene; 3: plants expressing GmRD22 gene; 4: plants expressing GFP.
Fig.11 depicts phenotypes of GmRD22, BnLAS and GFP transfected tomato Blacony Red seedlings exposed to the PEG6000 treatment.
Fig. 12 shows *Lycopersicon esculentum* cv. Balcony Red seedlings transfected with GFP and BnLAS expressing PVX vectors after 10 days of withholding irrigation and following recovery (2h after watering). Plants were sprayed with agrobacteria 12 days post sowing and exposed to drought 12 days post spraying.

### DETAILED DESCRIPTION OF THE INVENTION

The processes of the invention have their main application in agriculture, i.e. on a large scale. Thus, these processes are generally carried with a plurality of plants. Accordingly, the processes are generally applied to a plurality of plants sown or planted e.g. on a farm field. Before carrying out the process of the invention, the sown or planted plants preferably do not contain an expressible gene that provides the plants with the abiotic stress tolerance provided in the processes of the invention. Notably, the plants are preferably not transgenic for such gene.

After having sown or planted the plants, a user which is typically a farmer may observe growth of the plants and the health state of the plants. Further, the user may observe environmental conditions such as the weather and/or the condition of the soil for determining whether abiotic stress conditions for the plants have developed, are developing or may develop. Determining whether abiotic stress conditions are present may involve taking samples from the soil and examining the soil. Alternatively or additionally, determining whether abiotic stress conditions are present may involve taking samples from the plants or from a fraction of said plants and examining the plants.

For determining drought stress or salt stress, one ore more soil samples may be examined for water content or salt content, respectively. In a preferred embodiment, the processes of the invention are used for improving the tolerance or resistance (these terms are used interchangeably herein) of the plants against drought. Thus, determining drought stress and the further development of such stress may involve following weather forecasts for assessing the probability of rain.

Determining drought stress conditions is known in the art, see e.g. Jones et al., Journal of Experimental Botany 58 (No. 2) 119-130, 2007; Idso, Agricultural Meteorology 27, 59-70, 1982; Shimada et al., Journal of Arid Land Studies 22-1, 251-254, 2012. Soil measures and/or plant measures may be used for determining or following a water status affecting the plants. The known methods may also be classified into direct methods and indirect methods. Among direct methods, measurement of the relative water content (RWC) of soil or plant material and direct measurement of the leaf water potential e.g. with a pressure chamber, or using of sap flow sensors may be mentioned. Among indirect methods, remote infrared technologies based on the evaluation of canopy temperature or remote spectrometric methods based on the measuring of canopy spectral reflectance may be mentioned. As water becomes limiting, crop temperatures rise because they cannot transpire enough water to keep themselves cool. Plant leaves open their stomata to admit carbon dioxide for photosynthesis and at the same time water vapor flows out of the leaf, which cools the leaf surface. When soil water becomes limiting, transpiration decreases, thus reducing the leaf cooling effect and causing the crop temperature to rise. All objects emit energy or radiation that is measured as their temperature. For crop canopies the temperature is usually measured with a thermal infrared thermometer. Infrared thermometers (IRT) are commercially available. Irmak et al., Agronomy Journal, 92, 1221-1227, 2000) describe the determination of crop water stress index (CWSI) for the purpose of irrigation timing, but may also be used for determining drought stress in plants for the present invention. Other measures for following the water status are turgor pressure or stromatal conduct, and water potential in soil or the plants. In order not to depend on absolute determinations of the water status measures mentioned above, one may follow the water status continuously or regularly for easily recognizing development of drought stress.

At a desired growth state of the plant(s) or when the user has determined that resistance of the plants against an abiotic stress should be improved, the plants or parts thereof are provided with a suspension containing cells of an *Agrobacterium* strain comprising a nucleic acid molecule comprising a nucleic acid construct containing a nucleotide sequence of interest. The nucleotide sequence of interest can, upon expression in the plants, provide the plants with increased tolerance against the abiotic stress.

Also disclosed is a process of producing plants or parts of said plants, comprising the following steps:
(A) growing a plurality of plants on a field;
(B) determining whether drought stress affects said plants;
(C) if it was determined in step (B) that drought stress affects or may affect said plants, providing aerial parts of said plants with a suspension containing cells of an Agrobacterium strain comprising a nucleic acid molecule comprising a nucleic acid construct containing a nucleotide sequence of interest, said nucleotide sequence of interest providing said plants with increased drought stress resistance upon expression in said plants;
(D) harvesting said plants or desired parts thereof; and
(E) if step (C) was performed, using the harvest of step (D) for a purpose for which having performed step (C) is acceptable; or, if step (C) was not performed, using the harvest of step (D) for a purpose for which having performed step (C) is not acceptable.

A purpose for which performing step (C) may not be acceptable is food production for human consumption. A purpose for which performing step (C) may be acceptable is animal feed production or production of biofuel. Thus, the invention provides a crop production system and method for entire growing seasons, that strongly improves overall efficiency. On the one hand, loss of crop or plant material due to drought can be reduced, and the saved crops or biomass can be used for purposes for which genetic modification is not of concern. On the other hand, in seasons where no measures for increasing the drought tolerance are required due to favourable weather conditions, the plants grown are non-genetically modified, which is preferred by some consumers or in some countries.

In the present invention, the nucleotide sequence of interest may encode a protein capable of providing said plants with increased abiotic stress resistance such as drought resistance. Alternatively, the nucleotide sequence of interest may produce, upon expression, an RNA that may provide the plants with the increased abiotic stress resistance.

The nucleic acid molecule is generally a DNA molecule. In this case, the nucleotide sequence of interest contained therein is DNA sequence of interest.

In the processes of the invention, parts of said plants are transiently transfected with said nucleic acid molecule (vector) for transient expression of said nucleotide sequence of interest. This has the advantage that a decision on when the nucleotide sequence of interest should be expressed in plants, and which nucleotide sequence of interest to be expressed, can be delayed until shortly before the point of transfection. The term "transient" means that no selection methods are used for selecting cells or plants transfected with the nucleic acid molecule (vector) or with the nucleic acid construct from non-transfected cells or plants using, e.g. a selectable agent and a selectable marker gene capable of detoxifying the selectable agent. As a result, the transfected nucleic acid is generally not stably introduced into plant chromosomal DNA. Instead, transient expression methods make use of the effect of transfection in the very plant cells transfected.

A possible way of achieving expression of said nucleotide sequence of interest is the use of self-replicating (viral) replicons containing a nucleotide sequence encoding said nucleotide sequence of interest. Plant viral expression systems have been described in many publications both for the use of DNA viral vectors and for the use of RNA viral vectors, such as in WO2008028661, WO2006003018, WO2005071090, WO2005049839, WO2006012906, WO0210'f006 or WO020fi8fi64 and many more publications are cited in these documents.

Various methods for introducing a nucleic acid molecule, such as a DNA molecule, into a plant or plant part for transient expression are known. In the invention, Agrobacteria are preferably used for transfecting plants with the nucleic acid molecule (vector) or nucleic acid construct e.g. by agroinfiltration. A system and method for large scale infiltration of plants by agrobacteria is described in WO2009095183. In another embodiment, plants or plant parts are sprayed with a suspension containing cells of an *Agrobacterium* strain, which is well suitable for large scale applications to many plants such as to plants on a farm field. Such spray transfection processes are described in detail in WO20121019660.

The *Agrobacterium* strain may belong to the species *Agrobacterium tumefaciens* or *Agrobacterium rhizogenes* that are commonly used for plant transformation and transfection and which are known to the skilled person from general knowledge. The *Agrobacterium* strain to be used in the processes of the invention comprises a DNA molecule (Ti-plasmid) as said nucleic acid molecule, comprising a nucleic acid construct containing a DNA sequence of interest. The DNA sequence of interest may encode one or more than one protein to be expressed in plants. The nucleic acid construct is typically present in T-DNA of Ti-plasmids for introduction of the nucleic construct into plant cells by the secretory system of the *Agrobacterium* strain. On at least one side or on both sides, the nucleic acid construct is flanked by a T-DNA border sequence for allowing transfection of said plant(s) and introduction into cells of said plant of said nucleic acid construct. Preferably, said nucleic acid construct is present in T-DNA and flanked on both sides by T-DNA border sequences.

Most preferably, the nucleic acid construct is present in T-DNA of a Ti-plasmid of the *Agrobacterium* strain. Ti-plasmids may contain a selectable marker outside of said T-DNA for allowing cloning and genetic engineering in bacteria. However, the T-DNA that is transferred into cells of said plant preferably does not contain a selectable marker that would, if present, allow selection of plant or plant cells containing said T-DNA. Examples of selectable marker genes that should, in this embodiment, not be present in T-DNA of the Ti-plasmid are an antibiotic resistance gene or a herbicide resistance gene. Since the processes of the invention use transient transfection and expression of said nucleotide sequence of interest (or said protein), the processes do not comprise a step of selecting for plant cells having incorporated the nucleic acid molecule of the invention by using such antibiotic resistance gene or a herbicide resistance gene. Accordingly, no antibiotic resistance gene or a herbicide resistance gene needs to be incorporated into said plants, whereby the probability of spreading such genes in the environment is low in the processes of the invention.

The nucleic acid construct comprises the nucleotide sequence of interest such that the latter is expressible in plant cells. For this purpose, the nucleotide sequence of interest may be, in said nucleic acid construct, under the control of a promoter active in plant cells. Preferably, the nucleotide sequence of interest encodes a protein capable of providing plants with increased abiotic stress tolerance. Examples of the nucleotide sequence of interest are a DNA sequence encoding a DNA viral replicon or an RNA viral replicon, or a gene to be expressed. The gene provides, upon expression, the plant with the increased abiotic stress tolerance. Preferably, the gene encodes a protein capable of providing the plant with increases abiotic stress tolerance. Also the viral replicons encode such protein to be expressed in cells of the plant(s). The nucleic acid construct may comprise, in addition to the nucleotide sequence of interest, other sequences such as regulatory sequences for expression of the nucleotide sequence of interest, such as a transcription promoter and terminator. The nucleic acid construct may comprise a further gene to be expressed, e.g. a gene encoding a suppressor of gene silencing such as the P19 protein. Expression of such further gene may be under the control of the same or a different promoter as the promoter used for expressing the protein of the invention. *Agrobacterium*-mediated gene transfer and vectors therefor are known to the skilled person, e.g. from the references cited above or from text books on plant biotechnology such as Slater, Scott and Fowler, Plant Biotechnology, second edition, Oxford University Press, 2008.

As used herein, the term "promoter active in plant cells" means a DNA sequence that is capable of controlling (initiating) transcription in a plant cell. This includes any promoter of plant origin, but also any promoter of non-plant origin which is capable of directing transcription in a plant cell, i.e., certain promoters of viral or bacterial origin such as the cauliflower mosaic virus 35S promoter (CaMV35S promoter) (Harpster et al. (1988) Mol Gen Genet. 212(1):182-90, the subterranean clover virus promoter No 4 or No 7 (WO9606932), or T-DNA gene promoters but also tissue-specific or organ-specific promoters including but not limited to seed-specific promoters (e.g., WO89/03887), organ-primordia specific promoters (An et al. (1996) Plant Cell 8(1):15-30), stem-specific promoters (Keller et al., (1988) EMBO J. 7(12): 3625-3633), leaf specific promoters (Hudspeth et al. (1989) Plant Mol Biol. 12: 579-589), mesophyl-specific promoters (such as the light-inducible Rubisco promoters), root-specific promoters (Keller et al. (1989) Genes Dev. 3: 1639-1646), tuber-specific promoters (Keil et al. (1989) EMBO J. 8(5): 1323-1330), vascular tissue specific promoters (Peleman et al. (1989) Gene 84: 359-369), stamen-selective promoters (WO 89/10396, WO 92/13956), dehiscence zone specific promoters (WO 97/13865) and the like. For transient expression, constitutive promoters, i.e. promoters that are not developmentally regulated, are preferably used. However, constitutive promoters may be tissue-specific or organ-specific. Preferred promoters are those used in the Examples described below.

Herein, the term "construct" means a recombinant construct comprising a nucleotide sequence of interest. Preferably, the construct encodes at least a protein capable of providing said plants with increased abiotic stress resistance.

In embodiments wherein strong expression of the protein is desired, the nucleic acid construct may encode a viral vector that can replicate in plant cells to form replicons of the viral vector. In order to be replicating, the viral vector and the replicons contain an origin of replication that can be recognized by a nucleic acid polymerase present in plant cells, such as by the viral polymerase expressed from the replicon. In case of RNA viral vectors (referred to as "RNA replicons"), the replicons may be formed by transcription under the control of a promoter active in plant cells, from the DNA construct after the latter has been introduced into plant cell nuclei. In case of DNA replicons, the replicons may be formed by recombination between two recombination sites flanking the sequence encoding the viral relicon in the DNA construct, e.g. as described in WO00/17365 and WO 99/22003. If the replicon is encoded by the DNA construct, RNA replicons are preferred. Use of DNA and RNA viral vectors (DNA or RNA replicons) has been extensively described in the literature over the years. Some examples are the following patent publications: WO2008028661, WO2007137788, WO 2006003018, WO2005071090, WO2005049839, WO02097080, WO02088369, WO02068664. An example of DNA viral vectors are those based on geminiviruses. For the present invention, viral vectors or replicons based on plant RNA viruses, notably those based on plus-sense single-stranded RNA viruses may be used. Accordingly, the viral replicon may be a plus-sense single-stranded RNA replicon. Examples of such viral vectors those based on tobacco mosaic virus (TMV) and potex virus X (PVX). "Based on" means that the viral vector uses the replication system such as the replicase and/or other proteins involved in replication of these viruses. Potexvirus-based viral vectors and expression systems are described in EP2061890 or WO2008/028661. Many other plant viral replicons are described in the patent publications mentioned above.

In one embodiment, the processes of the invention comprise spraying aerial parts of said plant with a suspension containing cells of an *Agrobacterium* strain comprising a DNA molecule comprising a nucleic acid construct containing a DNA sequence of interest preferably encoding said protein.

The suspension that may be used for providing aerial parts of said plant is a liquid composition comprising *Agrobacterium* cells suspended in the liquid. The suspension is generally aqueous, which means that the liquid contains at least 50, preferably at least 80, more preferably at least 90 % by weight water. Other components in the liquid may be other liquid components compatible with the maintenance of the *Agrobacterium* cells in a viable form, which may be polar solvents such as polyhydric alcohols or dimethylsulfoxide. Further components may be nutrients, minerals or salts, buffering agents or spray adjuvants (see further below).

The suspension used for spraying plants or plant parts in the process of the invention may have a concentration of *Agrobacterium* cells of at most 1.1·10⁹ cfu/ml, which corresponds approximately to an *Agrobacterium* culture in LB-medium of an optical density at 600 nm of 1. Due to the high transfection efficiency achievable, notably if spraying is done with the use of an abrasive and/or a surfactant or wetting agent (see below), much lower concentrations may, however, be used, which allows treatment of many plants such as entire farm fields without the need for huge fermenters for *Agrobacterium* production. Thus, the concentration may be at most 2.2·10⁷ cfu/ml, preferably at most 1.1·10⁷ cfu/ml, more preferably at most 4.4·10⁶ cfu/ml. In one embodiment, the concentration is at most 1.1·106 cfu/ml of the suspension. For avoiding determination of cell concentrations in terms of cfu/ml, concentrations of agrobacterial suspensions are frequently assessed by measuring the apparent optical density at 600 nm using a spectrophotometer. Herein, the concentration of 1.1·10⁷ cfu/ml corresponds to a calculated optical density at 600 nm of 0.01, whereby the calculated optical density is defined by a 100-fold dilution with water or buffer of a suspension having an optical density of 1.0 at 600 nm. Similarly, the concentrations of 4.4·10⁶ cfu/ml and 1.1·106 cfu/ml correspond to a calculated optical density at 600 nm of 0.004 and 0.001, respectively, whereby the calculated optical densities are defined by a 250-fold or 1000-fold, respectively, dilution with water or buffer of a suspension having an optical density of 1.0 at 600 nm.

The suspension containing cells of an *Agrobacterium* strain used for spraying the plants or parts of a plant may contain at least one abrasive suspended in said suspension for improving transfection efficiency. The abrasive that may be used is a particulate material that is essentially insoluble in the aqueous suspension of *Agrobacterium* cells. The abrasive is believed to weaken, notably if used together with a wetting agent, the surface of plant tissue such as leaves, and thereby facilitates penetration of *Agrobacterium* cells into the intercellular space of plant tissue. As a result, the transfection efficiency is high.

The particulate material to be used as the abrasive of the invention may be carrier material as commonly used as carriers in wettable powder (WP) of pesticide formulations. In the context of wettable powders, these carriers are also referred to in the field of pesticide formulations as "fillers" or "inert fillers". Wettable powder formulations are part of the general knowledge in the field of plant protection. Reference is made to the handbook PESTICIDE SPECIFICATIONS, "Manual for Development and Use of FAO and WHO Specifications for Pesticides", edited by the World Health Organisation (WHO) and the FOOD and Agriculture Organization of the United States, Rome, 2002, ISBN 92-5-104857-6. Wettable powder formulations for plant protection are for example described in EP 1810569, EP1488697, EP1908348 and EP0789510. The abrasive may be a mineral material, typically an inorganic material. Examples of such carrier materials are diatomaceous earth, talc, clay, calcium carbonate, bentonite, acid clay, attapulgite, zeolite, sericite, sepiolite or calcium silicate. It is also possible to use quartz powder such as the highly pure quartz powder described in WO021087324. Preferred examples are silica, such as precipitated and fumed hydrophilic silica, and carborundum. The abrasive properties of diluents or fillers such as silica used in wettable powders are known (see "Pesticide Application Methods" by G.A. Matthews, third edition, Blackwell Science, 2000, on page 52 thereof).

As commercial products of particulate inorganic materials for use as abrasives, the hydrophilic silica Sipernat™ 22S and Sipernat™ 50 S, manufactured by Evonic Degussa may be mentioned. Other products are "Hi-Sil™ 257", a synthetic, amorphous, hydrated silica produced by PPG Industries Taiwan Ltd. or "Hubersorb 600™", a synthetic calcium silicate, manufactured by Huber Corporation. A commercial sub-micron sized silica is Hi-Sil™ 233 (PPG Industries) having an average particle size of around 0.02 µm.

The abrasive may have a median particle size between 0.01 and 40, preferably between 0.015 and 30, more preferably between 0.05 and 30, even more preferably between 0.1 and 30, even more preferably between 0.1 and 20, even more preferably between 0.5 and 20, and most preferably between 1.0 and 16 µm. In one embodiment, the median particle size is between 0.015 and 1 or between 0.02 and 0.5 µm. The median particle size is the volume median particle size that can be measured by laser diffraction using a Mastersizer™ from Malvern Instruments, Ltd. In order to avoid clogging of spraying nozzles, the maximum particle size of the largest particles contained in the abrasive should be at most 45 µm, preferably at most 40 µm, which may be determined by sieving. This condition is considered fulfilled, if the sieve residue is below 1.5 % by weight (following ISO 3262-19). The abrasive may have a D90 value of at most 40 µm, preferably of at most 30 µm, measured by laser diffraction as described above. Typically, the particle sizes above relate to primary particle sizes. The content of the abrasive in the aqueous suspension of the invention may be between 0.01 and 3, preferably between 0.02 and 2, more preferably between 0.05 and 1 and even more preferably between 0.1 and 0.5 % by weight of said suspension.

The suspension usable for spraying parts of the plants preferably contains an agricultural spray adjuvant. The spray adjuvant may be a surfactant or wetting agent. The surfactant or wetting agent has multiple advantages in the present invention. It reduces the surface tension of the water of the aqueous suspension and makes the waxy surface of plant leaves more permeable for agrobacteria. It further improves the stability of the suspension and reduces settling of the abrasive in the suspension. Surfactants used in the present invention are not particularly limited. However, nonionic surfactants are preferred. A measurement frequently used to describe surfactants is the HLB (hydrophilic/lipophilic balance). The HLB describes the ability of the surfactant to associate with hydrophilic and lipophilic compounds. Surfactants with a high HLB balance associate better with water soluble compounds than with oil soluble compounds. Herein, the HLB value should be 12 or greater, preferably at least 13. As noninionic surfactants, organo-silicone surfactants such as polyalkyleneoxide-modified heptamethyltrisiloxane are most preferred in the present invention. A commercial product is Silwet L77™ spray adjuvant from GE Advanced Materials.

*Agrobacterium* strains usable in the invention are those that are generally used in the art for transfecting or transforming plants. Generally, binary vector systems and binary strains are used, i.e. the *vir* genes required for transfer of T-DNA into plant cells on the one hand and the T-DNA on the other hand are on separate plasmids. Examples of usable *Agrobacterium* strains are given in the article of Hellens et al., Trends in Plant Science 5 (2000) 446-451 on binary *Agrobacterium* strains and vector systems. In the context of a binary *Agrobacterium* strain, the plasmid containing the *vir* genes is referred to as "vir plasmid" or "vir helper plasmid". The plasmid containing the T-DNA to be transfected is the so-called binary vector that is also referred to herein as "DNA molecule" or "vector". The term "strain" or *"Agrobacterium* strain" relates to components of the *Agrobacterium* other than the binary vector. Thus, herein, a binary *Agrobacterium* strain not containing a binary vector and after introduction of a binary vector are referred to by the same strain name.

The suspension of agrobacteria may be produced as follows. The DNA molecule or vector containing the nucleic acid construct may be transformed into the *Agrobacterium* strain and transformed *Agrobacterium* cultures are grown optionally under application of selective pressure for maintenance of said DNA molecule. In one method, the *Agrobacterium* strain to be used in the processes of the invention is then inoculated into a culture medium and grown to a high cell concentration. Larger cultures may be inoculated with small volumes of a highly concentrated culture medium for obtaining large amounts of the culture medium. Agrobacteria are generally grown up to a cell concentration corresponding to an OD at 600 nm of at least 1, typically of about 1.5. Such highly concentrated agrobacterial suspensions are then diluted to achieve the desired cell concentration. For diluting the highly concentrated agrobacterial suspensions, water is used. The water may contain a buffer or salts. The water may further contain the surfactant mentioned above. Alternatively, the concentrated agrobacterial suspensions may be diluted with water, and any additives such as the surfactant and the optional buffer substances are added after or during the dilution process. The abrasive may be added before, during or after dilution. It is however preferred to agitate the suspension during addition of the abrasive to uniformly disperse the abrasive in the agrobacterial suspension. The step of diluting the concentrated agrobacterial suspension may be carried out in the spray tank of the sprayer used for spraying the diluted suspensions.

The sprayer to be used for transfecting plants or plant parts in the process of the invention mainly depends on the number of plants or the area to be sprayed. For one or a small number of plants to be sprayed, pump sprayers as widely used in household and gardening can be used. These may have volumes of the spray tank of between 0.5 and 2 liters. For applications on a medium scale, manually operated hydraulic sprayers such as lever-operated knapsack sprayers or manually operated compression sprayers may be used. However, the high transfection efficiency achieved in the invention has its full potential in the transfection of many plants such as plants growing on a farm field or in a greenhouse. For this purpose, power-operated hydraulic sprayers such as tractor-mounted hydraulic sprayers equipped with spray booms can be used. Aerial application techniques using helicopters or airplanes are also possible for large fields. All these types of sprayers are known in the art and are described for example in the book "Pesticide Application Methods" by G.A. Matthews, third edition, Blackwell Science, 2000. In order to ensure a homogeneous suspension in the spray tanks of the sprayers, small or medium size sprayers may be shaken at regular intervals or continuously during spraying. Large sprayers such as the tractor-mounted sprayers should be equipped with an agitator in the spray tank.

Considering the presence of agrobacterial cells and optionally abrasive in the suspensions to be sprayed, sprayers used in the invention should produce spray of a droplet size at least of fine spray. Also, medium spray or coarse spray in the classification of sprays used in the above-mentioned book by G.A. Matthews, page 74, may be used. The main purpose of the spraying in the invention is wetting of plant tissue with the suspension. Thus, the exact droplet size is not critical. However, the transfection efficiency may be further improved by providing the spray to plant surfaces with increased pressure.

It is possible to apply the processes of the invention to transgenic plants containing a gene providing the plant with any desired trait other than the improved abiotic stress resistance trait provided in the processes of the present invention.

The invention also provides a composition containing cells of an *Agrobacterium* strain, said *Agrobacterium* strain comprising a heterologous DNA molecule comprising a nucleic acid construct containing a heterologous DNA sequence of interest operably linked to a promoter; said nucleotide sequence of interest providing a plant with increased abiotic stress resistance upon expression in said plant. The composition may be liquid or solid. Examples of solid compositions are dried or lyophilized compositions containing cells of said *Agrobacterium* strain. An example of a liquid composition is the suspension mentioned below. Lyophilized compositions may be prepared by freeze drying suspensions containing *Agrobacterium* cells, preferably in the presence of freeze-protection agents such as glycerol. The composition may further comprise a the spray adjuvant or the preferably non-ionic wetting agent such as an organosilicone surfactant mentioned above. The composition may be used for producing the suspension for spraying e.g. by dilution with water or an aqueous solvent.

In the process of the invention, said nucleotide sequence of interest or said protein capable of providing said plants with increased abiotic stress resistance may be expressed in both monocot and dicot (crop) plants. Common crop plants for the use in present invention include alfalfa, barley, beans, canola, cowpeas, cotton, corn, clover, lotus, lentils, lupine, millet, oats, peas, peanuts, rice, rye, sweet clover, sunflower, sweetpea, soybean, sorghum triticale, yam beans, velvet beans, vetch, wheat, wisteria, and nut plants. The plant species preferred for practicing this invention include, but not restricted to, representatives of Gramineae, Compositeae, Solanaceae and Rosaceae. Preferred plants are plants that do not normally enter the food chain such as *Nicotiana* species such as *N. tabacum* and *N. benthamiana* may be used.

Generally, the protein is expressed in the cytosol of cells of said plants or plant parts. In this case, no signal peptide directing the protein into a particular compartment is added to the enzyme.

The protein capable of providing said plants with increased abiotic stress resistance expressed and used in the present invention is generally a heterologous protein to the plant or plant part in which it is to be expressed in the invention, i.e. the plant or its part does not produce the protein naturally. It is possible to optimise codon usage of genes encoding these enzymes for expression in plants, notably in the plant employed for expressing them. Codon optimisation is a standard method for increasing expression yields. Codon-optimised genes and nucleic acids can be ordered from commercial sources such as Entelechon GmbH, Regensburg, Germany.

Increased abiotic stress resistance of a plant may be determined by treating a plant with the suspension of the invention and treating, as a control or reference, the same type of plant under otherwise identical conditions with a suspension not containing said cells of said *Agrobacterium* strain. Both plants are then subjected to identical conditions of abiotic stress. Increased abiotic stress resistance of the plant treated according to the invention can be detected by a more vigorous growth or better health condition.

Examples of abiotic stress resistances that may be increased are the following: drought resistance, salt resistance, osmotic stress resistance, cold resistance, freezing resistance, heat resistance, resistance to heavy metals, resistance to hypoxia or oxidative stress resistance.

Examples of nucleotide sequences of interest and proteins for improving drought stress tolerance in plants are given in the following. References to the genes and proteins are also given.

CspB from *Bacillus subtilis* subsp. subtilis. Str, such as JH642 (AAB01346). Castiglioni et al., Plant Physiology 147 (2008) 446-455 and Hunger et al., J. Bacteriol. 188 (2006) 240-248. The coding sequence of CspB codon-optimised for expression in plants is given SEQ ID NO: 1. The amino acid sequence is given as SEQ ID NO: 2.

E.coli cold shock protein 7.4 (cspA) gene. A coding sequence of the CspA protein suitable for expression in plants is given SEQ ID NO: 3. The amino acid sequence is given as SEQ ID NO: 4.

*Arabidopsis thaliana* nuclear transcription factor Y subunit B-1 (NF-YB1) (NM_129445.2) increases drought resistance in transgenic plants; cf. Nelson et al., PNAS 104 (2007) 16450-16455. A coding sequence is given SEQ ID NO: 5. An amino acid sequence is given as SEQ ID NO: 6.

BnLAS - GRAS family gene. Ectopic expression thereof in plants inhibits growth, delays flowering, delays leaf senescence and increases drought resistance, cf. Yang et al. (2011) Plant Cell Rep 30(3): 373-88. A coding sequence is given SEQ ID NO: 7. An amino acid sequence is given as SEQ ID NO: 8.

IPT (isopentenyl transferase) expression prevents the degradation of photosynthetic protein complexes during drought, cf. Rivero et al., Plant Cell Physiol. 51 (2010) 1929-1941, and Ori et al., The Plant Cell 11(1999) 1073-1080. An IPT coding sequence is given as SEQ ID NO: 21. An amino acid sequence is given as SEQ ID NO: 22.

BcWRKY46 - TF, a member of WRKY protein family, was reported to enhance the cold, salt and dehydration stress tolerance in tobacco, cf. Wang et al., Mol. Biol. Rep 39 (2012) 4553-4564. A coding sequence of BcWRKY46 is given SEQ ID NO: 11. An amino acid sequence is given as SEQ ID NO: 12.

HaHB1 from sunflower, AtHB13, AtPR2, AtPR4 and AtGLU - homeodomain-leucine zipper transcription factors confer tolerance to drought and salinity stress, cf. Cabello & Chan (2012) Plant Biotechnol. J. 10 (2012) 815-825. A complete coding sequence with removed splice sites is given SEQ ID NO: 13. An amino acid sequence is given as SEQ ID NO: 14.

Harpins from gram-negative plant pathogenic bacteria - stimulate hypersensitive cell death (HCD), pathogen defense and enhance growth in plants, drought tolerance, cf Zhang et al., J. Exp. Bot. 62 (2011) 4229-4238.

AVP1 H+ Pump (vacuolar H 1-pyrophosphatase) - plants overexpressing the vacuolar H1-pyrophosphatase are much more resistant to high concentrations of NaCl and to water deprivation than the isogenic wild-type strains; cf. Gaxiola et al. 2001 PNAS 98 (20) 11444-11449; Park et al., PNAS 102 (2005) 18830-18835; Zhang et al. (2011) Plant Signaling & Behavior 6: 6, 861-863. A coding sequence is given as SEQ ID NO: 17. An amino acid sequence is given as SEQ ID NO: 18.

Stress responsive gene SNAC1 (STRESS-RESPONSIVE NAC 1) from rice (GenBank: DQ394702) - SNAC1 (STRESS-RESPONSIVE NAC 1) significantly enhances drought resistance in transgenic rice in the field under severe drought stress conditions at the reproductive stage while showing no phenotypic changes or yield penalty, cf. Hu et al. (2006) PNAS 103: 12987-12992. A coding sequence is given as SEQ ID NO: 19. An amino acid sequence is given as SEQ ID NO: 20.

Salt tolerance may be increased by expressing in plants the following proteins:
GmRD22 (apoplast-localized BURP-domain protein from soybean) increases salinity and osmotic stress tolerance in transgenic Arabidopsis and rice, increases lignin production, cf. Wang et al. (2012) Plant Cell Environ. 35 (2012) 1932-1947. A coding sequence of GmRD22 is given SEQ ID NO: 9. An amino acid sequence is given as SEQ ID NO: 10.

HaHB1 from sunflower, AtHB13, AtPR2, AtPR4 and AtGLU - homeodomain-leucine zipper transcription factors as mentioned above can be used for increasing salt tolerance of plants.

Suppressed expression of the apoplastic ascorbate oxidase gene increases salt tolerance in plants, cf. Yamamoto et al. J. Exp Botany 56 (2005) 1785-1796. A coding sequence of the gene from *N. tabacum* is given SEQ ID NO: 15. An amino acid sequence is given as SEQ ID NO: 16. Expression of ascorbate oxidase may be suppressed by using RNA interference as is known in the art.

AVP1 H+ Pump (vacuolar H 1-pyrophosphatase) - Transgenic plants overexpressing the vacuolar H1-pyrophosphatase are much more resistant to high concentrations of NaCl and to water deprivation than the isogenic wild-type strains; cf. Gaxiola et al. 2001 PNAS 98 (20) 11444-11449; Zhang et al. (2011) Plant Signaling & Behavior 6: 6, 861-863; for sequences see above.

Stress responsive gene SNAC1 (STRESS-RESPONSIVE NAC 1) from rice (GenBank: DQ394702) - SNAC1 (STRESS-RESPONSIVE NAC 1) enhances drought salt tolerance in transgenic rice in the field under severe drought stress conditions, cf. Hu et al. (2006) PNAS 103: 12987-12992. A coding sequence is given as SEQ ID NO: 19. An amino acid sequence is given as SEQ ID NO: 20.

Cold tolerance may be increased by expressing in plants the following protein:
BcWRKY46 - TF, the member of WRKY protein family. Constitutive expression in transgenic tobacco reduced susceptibility to cold, ABA, salt and dehydration stress. Wang et al. (2012) Mol Biol Rep.

The SNAC1-targeted gene OsSRO1c modulates oxidative stress tolerance by regulating hydrogen peroxide in rice; cf. You et al., J. Experimantal Botany (2013).

LeCYP1 - Tomato Cyclophilin. A coding sequence is given as SEQ ID NO: 23. An amino acid sequence is given as SEQ ID NO: 24. Drought resistance though improved root formation;cf. Oh et al. (2006) Planta 224: 133-144.

In order to provide plants with increased abiotic stress resistance according to the present invention, any of the proteins of SEQ ID NO: 2, 4, 6, 8, 10, 12, 14, 16, 18, 20, 22 and 24 may be expressed in the plants. The same aim may be achieved by expressing in said plants a protein comprising an amino acid sequence having a sequence identity to any of the sequences of SEQ ID NO: 2, 4, 6, 8, 10, 12, 14, 16, 18, 20, 22, and 24 of less than 100%. The protein to be expressed may be a variant protein having an amino acid sequence having at least 80% sequence identity to the entire amino acid sequence of any one of SEQ ID NO: 2, 4, 6, 8, 10, 12, 14,16, 18, 20, 22, and 24. The amino acid sequence identity may be at least 85%, preferably at least 90%, more preferably at least 95% and even more preferably at least 97% to any one of amino acid sequence of any one of SEQ ID NO: 2, 4, 6, 8, 10, 12, 14, 16, 18, 20, 22, and 24. In calculating percent amino acid sequence identity, two sequences are aligned optimally and the number of identical matches of nucleotides or amino acid residues between the two sequences is determined. The number of identical matches is divided by the length of the aligned region (i.e., the number of aligned amino acid residues) and multiplied by 100 to arrive at a percent sequence identity value. A gap, i.e. a position in an alignment where a residue is present in one sequence but not in the other is regarded as a position with non-identical residues. Amino acid sequence identities may be determined using BLASTX 2.2.14 using the standard settings. The standard settings allow, for example, for sequence gaps in alignments.

The length of the aligned region is preferably over the entire length of the respective protein of SEQ ID NO: 2, 4, 6, 8, 10, 12, 14, 16, 18, 20, 22, or 24.

In another embodiment, the protein to be expressed comprises or consists of an amino acid sequence of at least 80 %, preferably at least 90 %, more preferably at least 95% of the number of amino acid residues of any one of SEQ ID NO: 2, 4, 6, 8, 10, 12, 14, 16, 18, 20, 22, and 24, and a sequence identity of at least 90% to SEQ ID NO: 2, 4, 6, 8, 10, 12, 14, 16, 18, 20, 22, or 24, respectively, over such length. In another embodiment, the protein to be expressed comprises or consists of an amino acid sequence of at least 80 %, preferably at least 90 %, more preferably at least 95% of the number of amino acid residues of any one of SEQ ID NO: 2, 4, 6, 8, 10, 12, 14, 16, 18, 20, 22, and 24, and a sequence identity of at least 95% to SEQ ID NO: 2, 4, 6, 8, 10, 12, 14, 16, 18, 20, 22, or 24, respectively, over such length. In these embodiments, the variant protein is preferably still capable of providing the plants with increased abiotic stress resistance of the respective protein of SEQ ID NO: 2, 4, 6, 8, 10, 12, 14, 16, 18, 20, 22, or 24, respectively.

Alternatively, the variant protein may have, or consist of, an amino acid sequence having from 1 to several amino acid additions, substitutions or deletions compared to the amino acid sequence of any one of SEQ ID NO: 2, 4, 6, 8, 10, 12, 14,16, 18, 20, 22, and 24, wherein the variant protein is preferably still capable of providing said plants with increased abiotic stress resistance. The maximum number of amino acid additions, substitutions or deletions may be at most 20, preferably at most 15, more preferably at most 10, and even more preferably at most 5, whereby the total number of additions, substitutions and additions together determine the number of "amino acid additions, substitutions or deletions".

In other embodiments, the protein to be expressed has an amino acid sequence similarity of at least 90% compared to the amino acid sequence of any one of SEQ ID NO: 2, 4, 6, 8, 10, 12, 14, 16, 18, 20, 22, and 24 over the entire length of these SEQ ID NOs. Preferably, the sequence identity is at least 95%, more preferably at least 98% over the entire length of these SEQ ID NOs. In another embodiment, the protein to be expressed comprises or consists of an amino acid sequence of at least 80 %, preferably at least 90 %, more preferably at least 95% of the number of amino acid residues of any one of SEQ ID NO: 2, 4, 6, 8, 10, 12, 14, 16, 18, 20, 22, and 24, and a sequence similarity of at least 90%, preferably at least 95%, more preferably at least 98% to any one of SEQ ID NO: 2, 4, 6, 8, 10, 12, 14, 16, 18, 20, 22, and 24, respectively, over such length.

Sequence similarity of protein sequences uses a substitution matrix with scores for all possible exchanges of one amino acid with another. The degree of similarity between protein sequences can, for example, be evaluated with the BLOSUM 62 substitution matrix (Henikoff and Henikoff, 1992, Proc. Natl. Acad. Sci. U. S. A.89:10915).

The protein capable of providing said plants with increased drought stress resistance may have a cold shock domain, preferably said protein is an RNA chaperone having a binding site for single-stranded RNA. RNA chaperones such as cold shock proteins are believed to resolve misfolded RNA molecules by destabilizing RNA duplexes or binding to single-stranded nucleic acids (see e.g. Hunger et al., J. Bacteriol. 188 (2006) 240-248). Preferably, the protein is or is a variant (as defined above) of *B*. *subtiliis* CspB of SEQ ID NO: 2. In one embodiment, the protein comprises the amino acid sequence of SEQ ID NO: 2.

Where the protein is a variant of the protein defined by the amino acid sequence of SEQ ID NO:2, said protein has at least the amino acid residues of CspA from E. coli W11, F18, F20, F31, H33, and F34 at positions corresponding to these positions in CspA from E. coli. Positions corresponding to positions in CspA from E. coli can be determined by alignment and correspond to an optimal alignment of the protein sequences.

In one embodiment, the protein is plant transcription factor LAS of SEQ ID NO: 8 or GmRE222 of SEQ ID NO: 10 or a variant thereof as defined above. In such embodiment, the plant may be a dicot plant, preferably a Solanaceae plant, and more preferably a tomato plant.

### EXAMPLES

### Reference Example 1: Determination of Agrobacterium cell concentration in liquid culture in terms of colony forming units (cfu)

The concentration of *Agrobacterium* cells in liquid suspension in terms of colony forming units per ml (cfu/ml) of liquid suspensions can be determined using the following protocol. Cells of *Agrobacterium tumefaciens* strain ICF 320 transformed with construct pNMD620 were grown in 7.5 ml of liquid LBS medium containing 25 mg/L kanamycin (AppliChem, A1493) and 50 mg/L rifampicin (Carl Roth, 4163.2). The bacterial culture was incubated at 28°C with continuous shaking. Absorbance or optical density of bacterial culture expressed in absorbance units (AU) was monitored in 1-ml aliquots of the culture using a spectrophotometer at 600 nm wavelength (OD600). The cell concentration estimated as a number of colony-forming units per milliliter of liquid culture (cfu/ml) can be analyzed at OD600 values 1; 1.3; 1.5; 1.7 and 1.8. For this purpose 250-µl aliquots of liquid culture were diluted with LBS-medium to achieve a final volume of 25 ml (dilution 1:100). 2.5 ml of such 1:100 dilution were mixed with 22.5 ml of LBS to achieve the dilution 1:1000. Liquid culture dilutions 1:100; 1:1,000; 1:10,000; 1:100,000; 1:1,000,000; 1:10,000,000 and 1:100,000,000 were prepared similarly. Aliquots of last three dilutions were spread on agar-solidified LBS medium supplemented with 25 mg/L kanamycin and 50 mg/L rifampicin (250 µl of bacterial culture per plate of 90 mm diameter). Plating of aliquots for each dilution was performed in triplicate. After 2 days incubation at 28°C, bacterial colonies were counted for each plate. Plating of 1: 1,000,000 and 1:10,000,000 dilutions resulted in few hundred and few dozen colonies per plate, respectively. So far as dilution 1:100,000,000 provided just few colonies per plate, this dilution was not used for calculation of cell concentration. The cell concentration was estimated according to the formula: cfu/ml = 4 x number of colonies per plate x dilution factor.

For transforming cell concentrations as measured by absorbance measurements at 600 nm (in LB medium) and in terms of cell-forming units, the following relation is used herein: an OD600 of 1.0 corresponds to 1.1 x 10⁹ cfu/ml.

### LBS medium (liquid)

1% soya peptone (papaic hydrolysate of soybean meal; Duchefa, S1330)
0.5% yeast extract (Duchefa, Y1333)
1% sodium chloride (Carl Roth, 9265.2)
dissolved in water, and the is adjusted to pH 7.5 with 1M NaOH (Carl Roth, 6771.2)

To prepare the solid LBS medium, liquid LBS medium was supplemented with 1.5% agar (Carl Roth, 2266.2). Media were autoclaved at 121°C for 20 min.

### Example 1: Vectors used in the following examples

In this study we used standard transcriptional vectors based on 35S CaMV promoter as well as TMV- and PVX-based viral replicons with or without cell-to-cell movement ability. Plasmid maps for all five types of cloning vectors are represented Fig. 1. In all cases the gene of interest is insterted using Bsal cloning sites with catg and gatc (taag) 5' and 3' overhangs, respectively. pNMD2492 transcriptional vector was created on the basis of pICBV10, a pBIN19-derived binary vector (Marillonnet et al., 2004, 2006). It contains two expression cassettes inserted between T-DNA right and left borders. An expression cassette adjacent to the right border comprised CAMV 35S promoter, omega translational enhancer, Bsal cloning site for the insertion of gene of interest and the nos terminator (listed in sequential order). Expression cassette adjacent to the left border contained 35S promoter followed by omega translational enhancer, coding sequence of P19 suppressor of silencing from Tomato Bushy Stunt Virus (TBSV) (GenBank accession no. CAB56483.1) and ocs terminator.

TMV-based vector with cell-to cell movement ability pNMD035, TMV-based vector lacking cell-to cell movement ability pNMD661, PVX-based vector with cell-to-cell and systemic movement ability pNMD670 and PVX-based vector disabled for both systemic and cell-to cell movement pNMD694 have been already described in the patent "Process for transfecting plants".

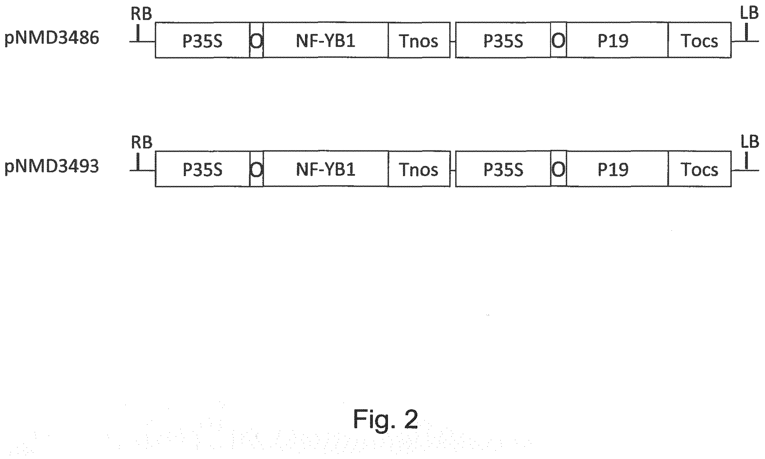

Plasmid construct pNMD3486 was created by direct insertion of coding sequence of *Arabidopsis thaliana* nuclear transcription factor Y subunit B-1 (NF-YB1) (GenBank: NM_129445.2) into pNMD1426 cloning vector using Bsal cloning site. Similarly, codon-optimized coding sequence of Cold shock protein CspB from *Bacillus subtilis* subsp. *subtilis* str JH642 (GenBank: AAB01346) was inserted into same cloning vector resulting in plasmid construct pNMD3493 (Fig. 2).

### Example 2: Influence of transient expression of NF-YB1 and CspB genes on the drought resistance of rapeseed plants.

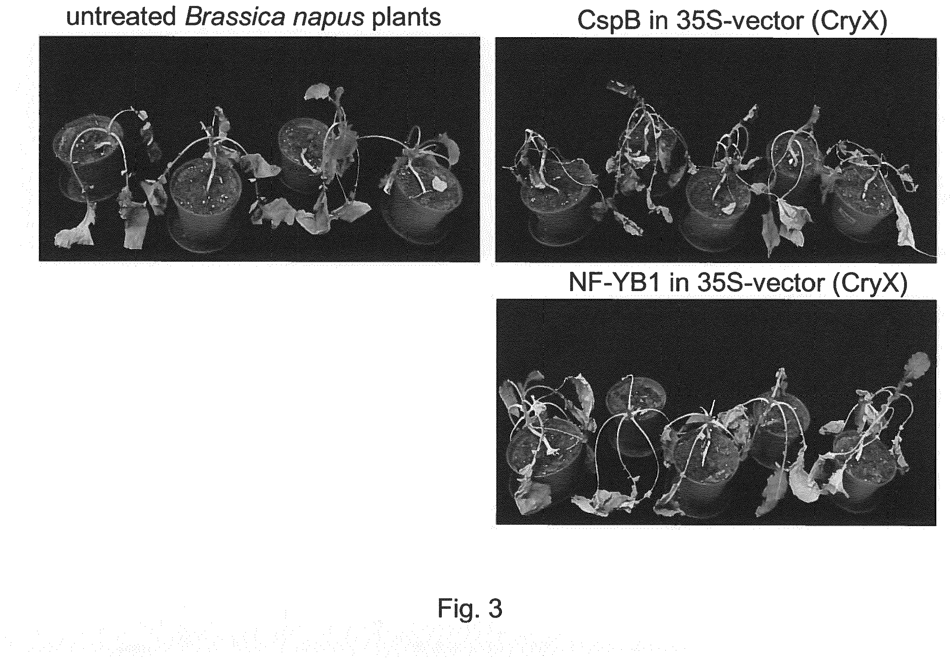

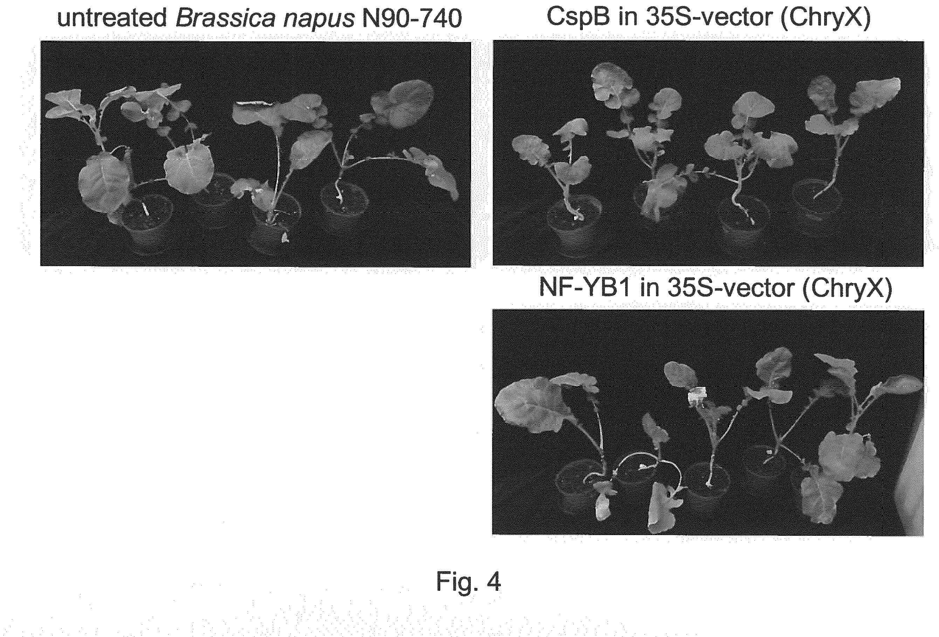

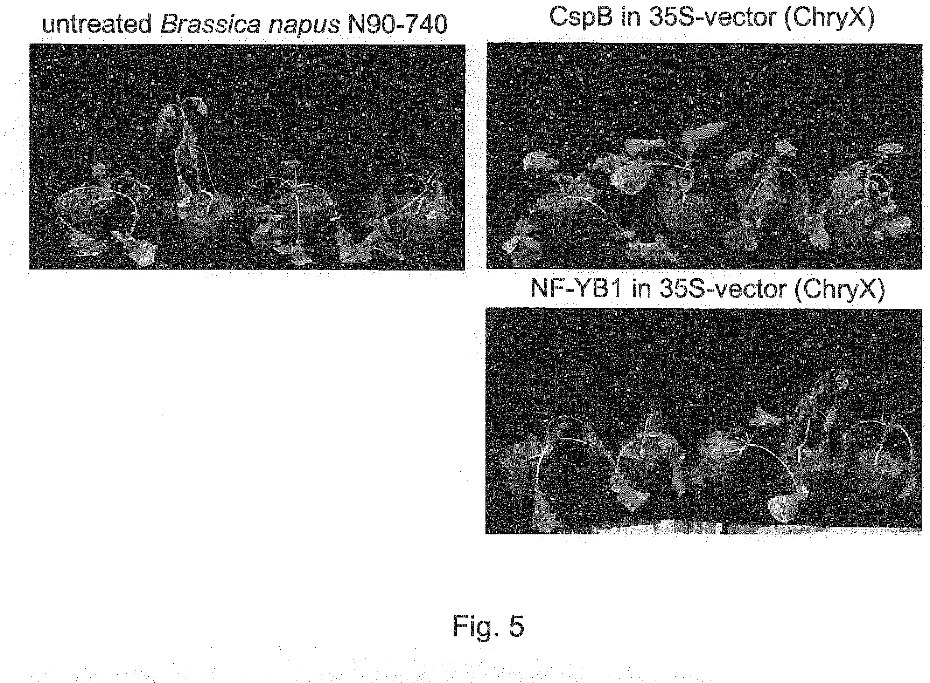

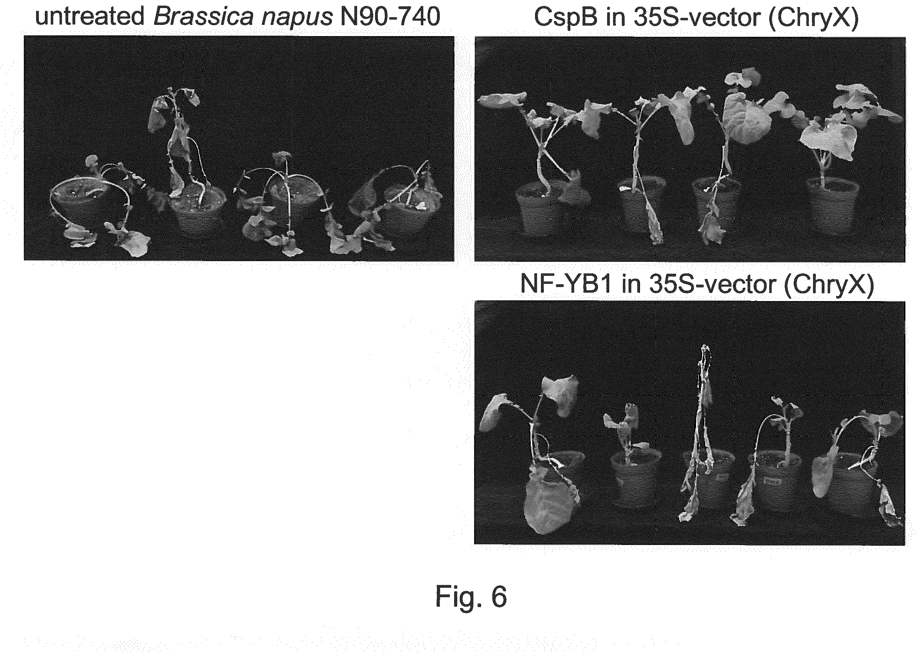

7 weeks old *Brassica napus* plants were sprayed with *Agrobacterium tumefaciens* CryX strain harboring pNMD3486 and pNMD3493 constructs (transcriptional vectors containing NF-YB1 and CspB coding sequences under the control of 35S promoter, respectively (Fig. 2). *A*. *tumefaciens* strain CryX is described in International Patent Application PCT/EP2013/000994. Watering of plants was stopped on the day of spraying. After 11 days of drought, plants were watered next 7 days. After that plants were subjected to 13 days of drought and watered again for 1 day. By the end of the first drought treatment (11 days) all NF-YB1, CspB and untreated control plants looked wilted without any significant difference between groups (Fig. 3). During next 7 days of watering all plants restored the shape they had before drought treatment, plants looked healthy and vigorous (Fig. 4). By the end the second drought treatment (8 days) CspB plants were slightly less wilted if compared with NF-YB1 plants (Fig. 5). After one day of re-watering CspB plants were much more vigorous than two other groups (Fig. 6).

### Example 3: Influence of transient expression of BnLAS and GmRD22 genes on the drought resistance of tomato plants.

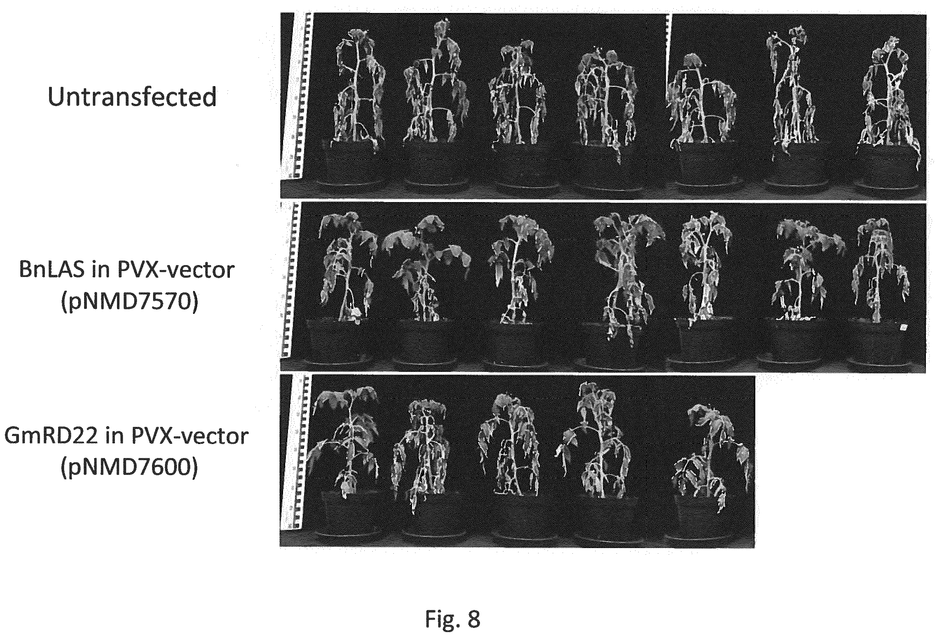

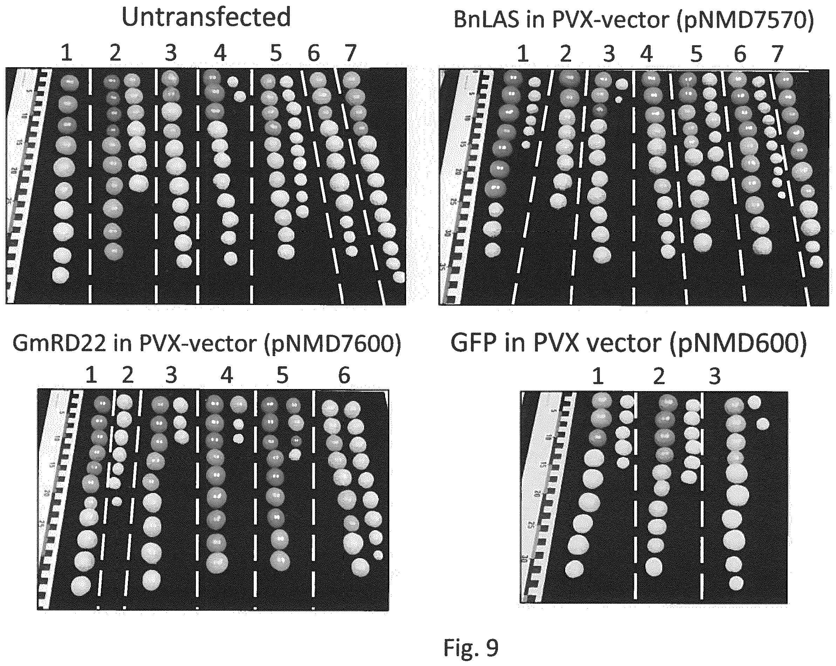

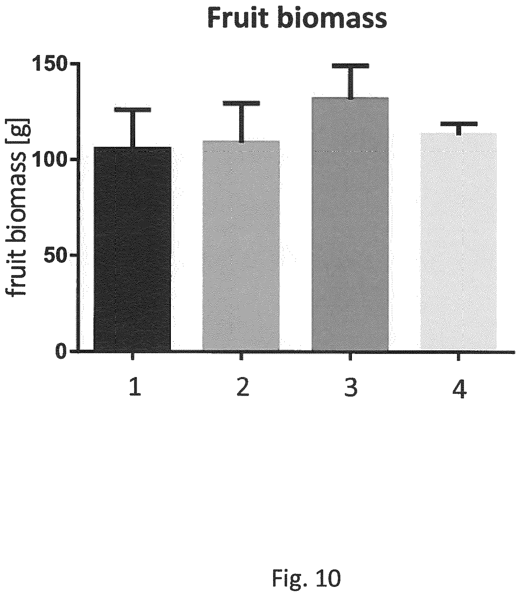

3 weeks old *Lycopersicon esculentum* cv Balcony Red plants were sprayed with *Agrobacterium tumefaciens* ICF320 strain harboring pNMD7570, pNMD7600 and pNMD600 constructs (PVX-based vectors with systemic movement ability containing BnLAS, GmRD22 and GFP coding sequences, respectively) (Fig. 7). Watering of plants was stopped 3 weeks post spraying. Drought exposure lasted for 14 days. By the end of drought treatment, BnLAS and GmRD22 transfected plants were less wilted than control untransfected plants (Fig. 8). After 14 days of drought (water saturation of soil approximately 34%), plants were watered again until the fruit harvest.
The fruit yield of tomato plants exposed to the drought was evaluated 100 days post spraying with agrobacteria. Fruits harvested for individual plants included in the experiment are shown in Fig. 9. After the drought exposure, GmRD22 transfected plants showed higher fruit biomass if compared with untransfected plants and plants transfected with BnLAS and GFP plants (Fig. 10).

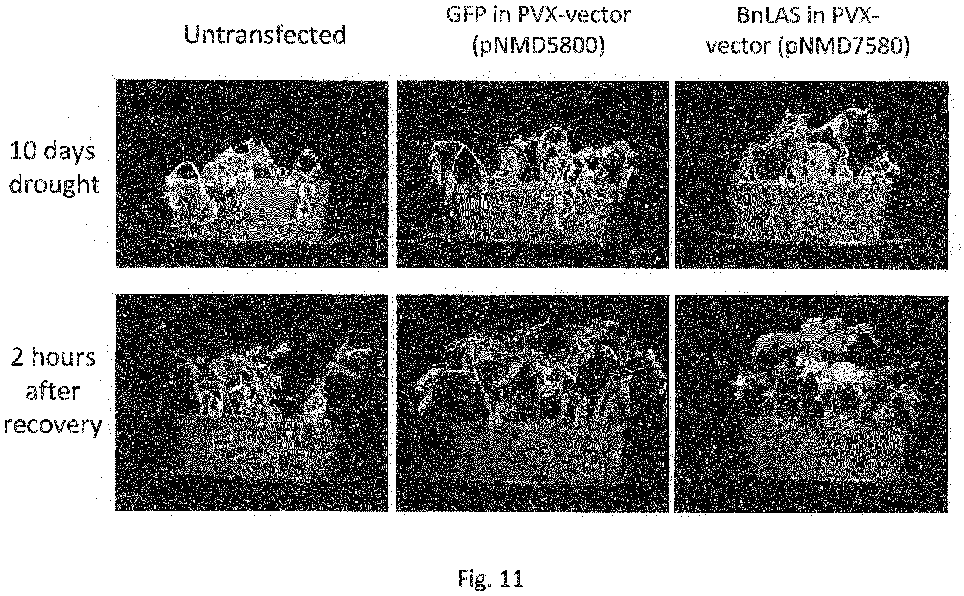

To evaluate the effect of transient expression of BnLAS on the drought resistance of young seedlings, 12 days old tomato (cv Balcony Red) plantlets grown in soil were sprayed with suspension of agrobacteria harboring PVX vectors pNMD7580 (BnLAS insertion) and pNMD5800 (GFP insertion, transfection control). 13 days post spraying, seedlings were exposed to 10 days drought followed by re-watering. After the 10 days drought treatment, BnLAS transfected plants were more vigorous and less wilted compared with untransfected seedlings and seedlings transfected with GFP vectors (Fig. 11, top panel). The difference is even more obvious after 2 hours of water recovery (Fig. 11, bottom panel).

### Example 4: Influence of transient expression of BnLAS and GmRD22 genes on the osmotic stress resistance of tomato plants.

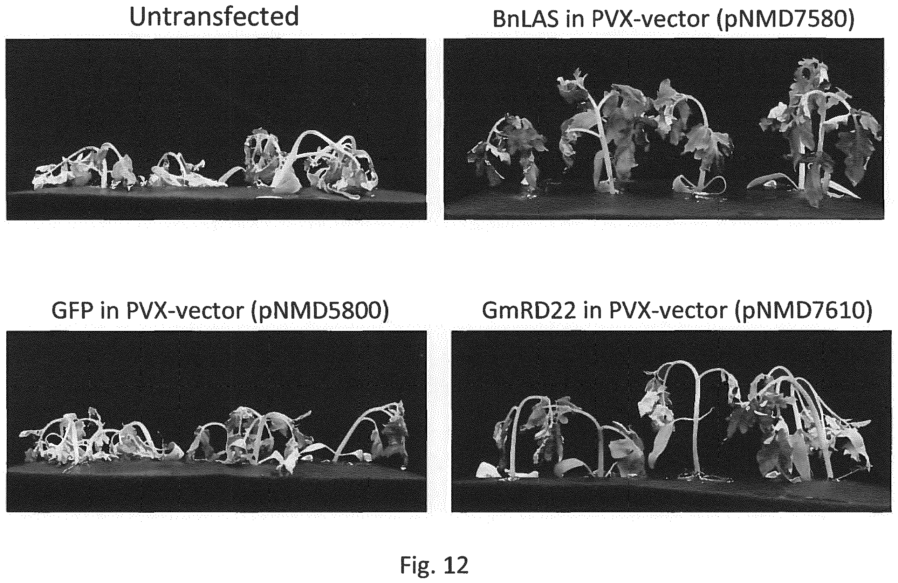

12 days old tomato (cv Balcony Red) seedlings grown in soil were sprayed with suspension of agrobacteria harboring PVX vectors pNMD7580 (BnLAS insertion), pNMD7610 (GmRD22 insertion) and pNMD5800 (GFP insertion, transfection control). 13 days post spraying seedlings were transferred into beakers containing 20% solution of PEG6000 and incubated there for 20 hours. As depicted in Fig. 12, BnLAS and GmRD22 transfected plants were more viable and less wilted when compared with untransfected plants and plants transfected with GFP vectors.

### Nucleic acid and amino acid sequences referred to herein

### CspB - cold shock protein from Bacillus subtilis

SEQ ID NO: 1: complete CDS, codon optimized for Brassica napus, synthesized by Entelechon, GenBank: AAB01346 
Atgctagagggcaaagtgaagtggttcaacagcgagaagggattcggctttatcgaagtggaaggcc
aggatgacgtgtttgttcacttctctgccatccaaggggaaggattcaagacactggaggaaggaca
ggcagtctcattcgagatagtcgaggggaatagaggacctcaagctgcgaacgttaccaaagaggct
tga
SEQ ID NO: 2: Amino acid sequence:
   MLEGKVKWFNSEKGFGFIEVEGQDDVFVHFSAIQGEGFKTLEEGQAVSFEIVEGNRGPQAANVTKEA

### E.coli cold shock protein 7.4 (cspA) gene, complete cds GenBank: M30139.1

SEQ ID NO: 3:CDS: 
Atgtccggtaaaatgactggtatcgtaaaatggttcaacgctgacaaaggcttcggcttcatcactc
ctgacgatggctctaaagatgtgttcgtacacttctctgctatccagaacgatggttacaaatctct
ggacgaaggtcagaaagtgtccttcaccatcgaaagcggcgctaaaggcccggcagctggtaacgta
accagcctgtaa
SEQ ID NO: 4: Protein:

Msgkmtgivkwfnadkgfgfitpddgskdvfvhfsaiqndgyksldegqkvsftiesgakgpaagnv
tsl

### NF-YB1 - Arabidopsis thaliana nuclear transcription factor Y subunit B-1

SEQ ID NO: 5: ORF original sequence, synthesized by Entelechon GenBank: NM_129445.2 
atggcggatacgccttcgagcccagctggagatggcggagaaagcggcggttccgttagggagcagg
atcgataccttcctatagctaatatcagcaggatcatgaagaaagcgttgcctcctaatggtaagat
tggaaaagatgctaaggatacagttcaggaatgcgtctctgagttcatcagcttcatcactagcgag
gccagtgataagtgtcaaaaagagaaaaggaaaactgtgaatggtgatgatttgttgtgggcaatgg
caacattaggatttgaggattacctggaacctctaaagatatacctagcgaggtacagggagttgga
gggtgataataaggggatcaggaaagagtggagatggatcaaatagagatgctggtggcggtgtttct
ggtgaagaaatgccgagctgg
SEQ ID NO: 6: Amino acid sequence

MADTPSSPAGDGGESGGSVREQDRYLPIANISRIMKKALPPNGKIGKDAKDTVQECVSEF
ISFITSEASDKCQKEKRKTVNGDDLLWAMATLGFEDYLEPLKIYLARYRELEGDNKGSGK
SGDGSNRDAGGGVSGEEMPSW

### BnLAS - Brassica napus cultivar Hua shuang No. 5 transcription factor LAS

SEQ ID NO: 7: Complete CDS, synthesized by Eurofins MWG Operon Splice sites removed: 454-456:AG→TC,C651T
   GenBank: HQ324233.1 
atgcttacttccttcaaatcctctagctcctcctccgaagatgccaccgagaatcctcctcctc
ctccgttatgcctcgcctcatcttctgccgcaacatccgccgctcatcacctccgtcgtctactatt
cacagctgcggatttcatctctcagtccaacgtctccgccgctcaaaacatactctcaatcctctcc
tcaaactcttccccttacggggactccacggagcggctcgtccatctcttcaccaaagccttgtccg
tacggatcggcttgtctgaaaacactgccacgtggacagcgaacgaaatggcttctagctccacggt
tttacaagcagtgtatgcaaagaacagttcttgtttcgaaccaagaacaacaactctgatctc
gagtcttgttactatctttggctgaaccaactaacaccgtttattcggttctcccatttaacggcga
accaagcgatcctcgacgcgactgagacaaacaacggtaacggagctttacatatacttgacttaga
tatatcacaaggacttcaatggcctccgttgatgcaagccctagccgagagatcatcatcaaaccct
agcagtactccacctccttccctccgcataaccggatgtggtcgagatgtaaccgtattaaaccgaa
ccggagatcggttaacccggtttgctaactctctaggtcttcagtttcagtttcacacgcttgtgat
cgctgaagaagacctcgccggacttttgcttcagatcagattattagctctctccgccgtacaagga
gagtccatcgccgtcaactgcgtccacttccttcacagattctttaacgacgacggagacatgatcg
gtcacttcctgtcggcgatcaagagcttaaaccctagaatcgtgacaatggcggagagagaagcgaa
ccatggagatccttcgttcttgactagattctcagaggctttagatcatttcatggcgatatttgat
tcgttggaagcgactttgccgccaaacagcaaagagaggctaaccctagagcaacggtggttcggta
tggagattttggatgttgtggcggcggaagcggcggagagaaagcaaagacatcggaggtttgaggt
ttgggaggagatgatgaagagacatggctttgctaacgtgccaataggaagctttgctttctctcaa
gctaagcttctgcttagactccattatccttcagaaggttataatcttcagtttctcaacgactctt
tgtttcttggatggaaaaatcgtcttctcttctccgtttcgtcgtggaaa
SEQ ID NO: 8: Amino acid sequence

MLTSFKSSSSSSEDATENPPPPPPLCLASSSAATSAAHHLRRLLFTAADFISQSNVSAAQNILSILS
SNSSPYGDSTERLVHLFTKALSVRIGLSENTATWTANEMASSSTVFTSSVCKEQFLFRTKNNNNSDL
ESCYYLWLNQLTPFIRFSHLTANQAILDATETNNGNGALHILDLDISQGLQWPPLMQALAERSSSNP
SSTPPPSLRITGCGRDVTVLNRTGDRLTRFANSLGLQFQFHTLVIAEEDLAGLLLQIRLLALSAVQG
ESIAVNCVHFLHRFFNDDGDMIGHFLSAIKSLNPRIVTMAEREANHGDPSFLTRFSEALDHFMAIFD

SLEATLPPNSKERLTLEQRWFGMEILDVVAAEAAERKQRHRRFEVWEEMMKRHGFANVPIGSFAFSQ
AKLLLRLHYPSEGYNLQFLNDSLFLGWKNRLLFSVSSWK

### GmRD22 - apoplast-localized BURP-domain protein from soybean

SEQ ID NO: 9: Complete CDS, synthesized by Eurofins MWG Operon (C843G silent mutation for BsaI site removal)
   GenBank: BT097299.1 
Atggagtatcgtctcctacccatttttactttactcaatcttgcactggtggcaatccatgctgctt
tacctcctgaagtttactggaagtcggtgcttcctactacgccaatgccaaaagccatcactgatat
cctttaccccgattgggtggaagagaaaagtacctcagtgaatgttggaggcaagggcgtaaacgtg
catgcaggaaaaggaggaggtggcaccaatgtcaacgttggtggaaaaggatcaggcggaggcgtga
acgtgcatgcaggtcacaagggaaagccagtgcatgtttctgttggctcaaagtctccattcaatta
catctacgcttcaacggagactcaattacacgatgaccccaacgtcgcactcttcttcttggaaaag
gacttgcatcccggaacaaagttgaacttgcacttcaccaccagttccaatattcaagccacattct
tgccacgccaagttgcggattctatacccttttcatccagcaaggtggaggttgtattcaacaagtt
ttccgtaaaacccgggtcagaggaggcccagatcatgaagaatactctcagtgagtgtgaagagggt
ggcatcaaaggagaggaaaagtactgtgccacttcgcttgaatccatgattgatttcagcacttcca
agcttggaaaaaatgttgaggttgtgtccacggaagtagtggaggacaaggaaacgggattgcagaa
atacaccgtagcaccgggagtgaacaagttatcaggggacaaggctgttgtgtgccacaagcagaac
taccccttatgctgtttttactgtcacaaaactgagacgacaagagcttactctgtgcctttggagg
gtgctaatggggttagggttaaagcggtagcagtgtgccacactcacacgtcggaatggaaccctaa
acatttggcctttcaagtgctcaaagttaagccaggaaccgttcctgtctgccacttcctacctgag
gatcatgttgtttgggttcccaag
SEQ ID NO: 10: Amino acid sequence

MEYRLLPIFTLLNLALVAIHAALPPEVYWKSVLPTTPMPKAITDILYPDWVEEKSTSVNVGGKGVNV
HAGKGGGGTNVNVGGKGSGGGVNVHAGHKGKPVHVSVGSKSPFNYIYASTETQLHDDPNVALFFLEK
DLHPGTKLNLHFTTSSNIQATFLPRQVADSIPFSSSKVEVVFNKFSVKPGSEEAQIMKNTLSECEEG
GIKGEEKYCATSLESMIDFSTSKLGKNVEVVSTEVVEDKETGLQKYTVAPGVNKLSGDKAVVCHKQN
YPYAVFYCHKTETTRAYSVPLEGANGVRVKAVAVCHTHTSEWNPKHLAFQVLKVKPGTVPVCHFLPE
DHVVWVPK

### BcWRKY46 - Brassica rapa subsp. chinensis transcriptional factor WRKY46

SEQ ID NO: 11: Complete CDS, synthesized by Eurofins MWG Operon. GenBank: HM585284 
Atggctatggaagagaaactcgtgatcaacgaactggaacaagggagagagcttgcccaacgtttga
tgagcaatctcaaagacacttcctcaatcgaatccagcaagaacttgatctctgagatcctcagtat
ctaccagaatgctatttccatgttagacgacaagaaggtccttaaacgtagccgtgagatcgatgac
aaagattctaagaacgtgataaaaaagaggcaagtgtttgagaagaagacagagaaagttagtttct
ttgtcggagcaggacaagaaaagggttccattgatgatggttattgctggagaaagtacggtcaaaa
agagattcatggatccattaatccaagaggatatttcagatgcacgcatcgattcacacagaactgt
ttagcagtgaagcaagtccaaaaatcagacagagatccttccatttcgaagtgaagtatgtcgga
gccacacttgtaacaacactactacgtccccaaagacaccgaacttctctatttcgatgttccaaca
agaagacatcaaaccgacgaaaacagaggaagcgatgatgagtcttgaagatctcgagagcactaag
aacattttcagaacgttttctttctccaactacgagattgagaatgctggtggttggaaaggca
acctcttccatgaggatcagctgtctcctgctgcgactacgtcagggtctggaatcaccagcgaggt
tgcaacagctcctgcttccgttgagaactcggagactgcagattcgtatttctcgtctttggacaat
attatcgactttggaccggattggttgctgtcgtgtgacgttttgaattgg
SEQ ID NO: 12: Amino acid sequence

MAMEEKLVINELEQGRELAQRLMSNLKDTSSIESSKNLISEILSIYQNAISMLDDKKVLKRSREIDD
KDSKNVIKKRQVFEKKTEKVSFFVGAGQEKGSIDDGYCWRKYGQKEIHGSINPRGYFRCTHRFTQNC
LAVKQVQKSDRDPSIFEVKYVGSHTCNNTTTSPKTPNFSISMFQQEDIKPTKTEEAMMSLEDLESTK
NIFRTFSFSNYEIENAGGGWKGNLFHEDQLSPAATTSGSGITSEVATAPASVENSETADSYFSSLDN
IIDFGPDWLLSCDVLNW

### HaHB1 - Helianthus annuus HD-Zip subfamily I trancription factor

SEQ ID NO: 13: Complete CDS, synthesized by Eurofins MWG Operon; splice sites removed G561A, G750A
   GenBank: HQ287802.1 
Atgacttgcactggaatggctttcttctcctccaatttcatgttacaatcctcccaagaagatgacc
atcatgcccctacatctctctctccaatcctcccaccttgcagtaccaccactcaagatttcagtgg
tgctgctttcttgggaaaaagatctatgtcttcttactcaggtttgaacaacaacaacatggatgga
tgtgatcaagaagggaacatgaatggagaagatgagttatcagatgatggatcacagcttcttgcag
gagagaaaaagaggagattaaacatggaacaagtgaagacacttgagagaaactttgagttaggaaa
taagcttgaacctgagaggaaaatgcaacttgcaagagcacttggactacaaccaagacagattgct
atatggtttcaaaacagaagagctagatggaaaactaaacagttggaaaaagactatgatgccctca
agagacagtttgaagctgttaaagctgagaatgattcactccaatctcaaaatcataaacttcatgc
tgagataatggcactaaaaaatagagagccagcagaactaatcaacctcaacataaaagaaacagaa
ggatcttgcagcaaccgaagcgaaaacagctctgaaatcaaactagacatctcaagaacaccggcta
ccgatagccctttatcatcacaccatcaacaccaacaccagccaatacctaatctttttccatcgtc
gaatatcgatagacctaattcgaataacattgtggcgcatcaactttccacaattcgtcatcaagg
ccggcagatcatcaacttcattgccacaaactcgatcaatcgaatgccattaaagaagaatgtttta
gcacaatgtttgttggtatggatgatcaatcagggttttggccatggttggaacaaccacaattcaa
t
SEQ ID NO: 14: Amino acid sequence

MTCTGMAFFSSNFMLQSSQEDDHHAPTSLSPILPPCSTTTQDFSGAAFLGKRSMSSYSGLNNNNMDG
CDQEGNMNGEDELSDDGSQLLAGEKKRRLNMEQVKTLERNFELGNKLEPERKMQLARALGLQPRQIA
IWFQNRRARWKTKQLEKDYDALKRQFEAVKAENDSLQSQNHKLHAEIMALKNREPAELINLNIKETE
GSCSNRSENSSEIKLDISRTPATDSPLSSHHQHQHQPIPNLFPSSNIDRPNSNNIVAHQLFHNSSSR
PADHQLHCHKLDQSNAIKEECFSTMFVGMDDQSGFWPWLEQPQFN

### NtAAO - Nicotiana tabacum ascorbate oxidase precursor

SEQ ID NO: 15: Complete CDS, synthesized by Eurofins MWG Operon. GenBank: D43624 
atggcttccttaggcttcttgttcttcttcttgttgccattgattttgcttgagttatcttcatcta
ggtcagtaatggcagcaaaaacacggcattttaaatggacgtggaatatattcattggtcaccaga
tggtgaagaaagtgtagtaatgggaatcaatggacagtttcctggtccaactattagggcaaaagct
ggtgatactgttgctgttcatcttactaacaagctacatactgaaggtgttgtcattcattggcatg
gaatccgacagatcggaacaccatgggctgatggaactgcagcaatttcccaatgcgccattaaccc
tggagagacatttctctataggtttaaagttgataaggcagggacatacttctaccatggacactat
ggaatgcaaagatcagcagggctatatggttcactaatagtggaagttggagaaggtgaaaaagaac
cattccattatgatggagaattcaatttattgcttagtgactggtggcacaaaggttcccatgaaca
agaagttgacctctcttccaatcctcttcgttggattggtgaaccccagacattgttgctaaatggg
agaggtcaatacaattgttcacttgcggcgcggtttagcaaaccaccacttccacagtgcaagttaa
gagggggtgaacagtacgcaccccagattctgcgcgtgcgtcccaacaagatttacaggcttagggt
ggcaagtactactgcattgggttcactcagcttggccattgggggtcacaagatggtggtagtagaa
gcagatggaaactatgttcaaccatttcagtacaagacatggacatttattcaggtgaaagctatt
cagtccttttcaaaacagatcaagatcctaccaaaaactattggatttcaataaatgtaagaggaag
agaaccaaaaacacctcaaggcctcaccttattaaactatcttccaaattctgcatccaaatttcca
 
actttaccaccacctatagcacccctttggaatgattataaccatagtaagtcattttctaacaaaa
tttttgccttaatgggatcacctaagccaccacctcagaaccatcgtcgtatcatcctgctcaatac
tcagaacaaaatcgatggttacacgaaatgggctataaataacgtgtcgttggtcttgccaacgcaa
ctttatttaggctcgattagatatggcataaacgcgtttgacacgaaacctccaccggacaacttcc
ctaaggactatgatgtcctaaaacaagcaccaaattctaattctacatatggtaatggtgtgtatat
gctaaagttcaatactacaattgacattatcctacaaaatgcaaatgccttagctaaagatgttagt
gaaattcatccttggcatttgcatggacatgattttgggtattgggatatggagaagggaaatta
gtgaaaaagatgtcaagaagttcaatttaaagaatccaccattgagaaatactgctgtgatttttcc
ctttggttggactgcactaagatttgtgacagataatcctggagtttgggcttttcattgtcatatt
gagccacatttacatatgggaatgggagttatatttgctgaaggtgttcatcttgtcaagaaaatac
ctaaagaagctttggcttgtggtttgacaggaaaatgttgatgagtaacaagcataat
SEQ ID NO: 16: Amino acid sequence

MASLGFLFFFLLPLILLELSSSRSVMAAKTRHFKWDVEYIHWSPDGEESVVMGINGQFPGPTIRAKA
GDTVAVHLTNKLHTEGVVIHWHGIRQIGTPWADGTAAISQCAINPGETFLYRFKVDKAGTYFYHGHY
GMQRSAGLYGSLIVEVGEGEKEPFHYDGEFNLLLSDWWHKGSHEQEVDLSSNPLRWIGEPQTLLLNG
RGQYNCSLAARFSKPPLPQCKLRGGEQYAPQILRVRPNKIYRLRVASTTALGSLSLAIGGHKMVVVE
ADGNYVQPFSVQDMDIYSGESYSVLFKTDQDPTKNYWISINVRGREPKTPQGLTLLNYLPNSASKFP
TLPPPIAPLWNDYNHSKSFSNKIFALMGSPKPPPQNHRRIILLNTQNKIDGYTKWAINNVSLVLPTQ
LYLGSIRYGINAFDTKPPPDNFPKDYDVLKQAPNSNSTYGNGVYMLKFNTTIDIILQNANALAKDVS
EIHPWHLHGHDFWVLGYGEGKFSEKDVKKFNLKNPPLRNTAVIFPFGWTALRFVTDNPGVWAFHCHI
EPHLHMGMGVIFAEGVHLVKKIPKEALACGLTGKMLMSNKHN

### AVP1_ARATH - Arabidopsis thaliana vacuolar H+ - pyrophosphatase (AVP-3)

SEQ ID NO: 17: Complete CDS, synthesized by Eurofins MWG Operon; (with E229D mutation; splices sites removed: A261G, C2004G, A2190T) GenBank: AEE29349.1 
atggtggcgcctgctttgttaccggagctctggacggagatccttgtaccgatttgtgcggtgattg
gtatcgccttttcgcttttccaatggtacgttgtatctcgcgtgaaactcacctctgacctcggcgc
atcgtcttccggtggagctaacaatggaagaatggatacggtgattatctaatcgaggaagaggaa
ggtgttaatgaccagagtgttgtcgctaagtgcgctgagattcagactgctatttccgagggtgcaa
cttcattcctattcacggagtacaaatatgttggtgtcttcatgatttctttgctgctgttatctt
tgttttcctcggctctgttgagggattcagcactgataacaagccttgtacttacgacaccaccaga
acctgcaagcctgcattggctactgcagctttcagtaccattgctttcgtgcttggtgctgttacct
ctgttctatctggtttccttgggatgaagattgctacatacgctaatgctaggaccactttggaggc
gaggaaaggtgttggaaaggcgttcattgttgcattcaggtctggtgctgtgatgggtttccttctt
gcagcgagtggtctattggtgctttacattactatcaatgtgttcaagatctattacggagatgact
gggaaggtctttttgacgctattactggttatggtcttggtgggtcttccatggctctctttggccg
tgttggtggtgggatctacactaaggctgctgatgtcggcgctgaccttgtcggtaaaattgagagg
aatattccagaggatgatccaagaaacccagctgtcattgctgataatgtcggtgacaatgttggtg
acattgctggtatgggatctgatctctttggatcatatgctgaagcatcatgcgctgctcttgttgt
tgcctcgatctcatctttcggaatcaaccacgacttcactgccatgtgctacccattgctcatcagt
tcaatgggaatcttggtttgtttgatcacaactctctttgccactgacttctttgagattaagcttg
tcaaggagattgaaccagcattgaagaaccagctcattatctcaactgttattatgactgttggtat
tgctattgtgtcatgggttggcttaccgacctcctttaccatcttcaactttggaacacaaaaagtt
gtcaagaactggcagctattcctttgtgtttgtgttggtctttgggctggactcattattggtttcg
tcactgagtactacactagtaacgcctacagccctgtgcaagatgttgcagattcatgcagaactgg
tgcagctaccaatgttatcttcggccttgctcttggttacaaatccgtcattattccaatctttgct
attgctatcagtatattcgttagcttcagctttgctgctatgtatggtgttgctgttgctgctcttg
gtatgctcagtaccattgccactggtttggcaattgatgcttatggtcccatcagtgacaatgctgg
tggtattgctgaaatggctggaatgagccaccgcatccgtgaaagaactgatgctcttgatgccgct
ggaaacaccactgctgctattggaaagggatttgccattggctctgctgccctagtctccttggctc
tctttggtgcctttgtgagccgtgcagggatccacaccgtagatgttttgacccctaaagttatcat
tgggctccttgttggtgccatgcttccttactggttctctgccatgacaatgaagagtgtgggaagt
 
gcagctcttaagatggttgaagaagttcgcaggcagttcaacaccatccctggacttatggaaggaa
ccgcaaaaccagactacgccacatgtgtcaagatctccaccgatgcttccatcaaggaaatgatacc
tcctggttgccttgtcatgctcacacctctcattgttggtttcttctttggagttgagacgctctct
ggtgtcctcgccggatctcttgtatccggtgttcagatcgccatatcagcatctaacactggtggtg
cctgggacaacgccaagaaatacatcgaggctggtgtatcagagcacgcaaagagccttggaccaaa
gggttcagagccacacaaggcagctgtgattggagacacaattggtgacccattgaaggatacttca
ggaccttcattgaacatcctcatcaagctcatggctgttgagtctcttgtctttgctcccttcttcg
ccactcacggtggtatccttttcaagtacttc
SEQ ID NO: 18: Amino acid sequence

MVAPALLPELWTEILVPICAVIGIAFSLFQWYVVSRVKLTSDLGASSSGGANNGKNGYGDYLIEEEE
GVNDQSVVAKCAEIQTAISEGATSFLFTEYKYVGVFMIFFAAVIFVFLGSVEGFSTDNKPCTYDTTR
TCKPALATAAFSTIAFVLGAVTSVLSGFLGMKIATYANARTTLEARKGVGKAFIVAFRSGAVMGFLL
AASGLLVLYITINVFKIYYGDDWEGLFDAITGYGLGGSSMALFGRVGGGIYTKAADVGADLVGKIER
NIPEDDPRNPAVIADNVGDNVGDIAGMGSDLFGSYAEASCAALVVASISSFGINHDFTAMCYPLLIS
SMGILVCLITTLFATDFFEIKLVKEIEPALKNQLIISTVIMTVGIAIVSWVGLPTSFTIFNFGTQKV
VKNWQLFLCVCVGLWAGLIIGFVTEYYTSNAYSPVQDVADSCRTGAATNVIFGLALGYKSVIIPIFA
IAISIFVSFSFAAMYGVAVAALGMLSTIATGLAIDAYGPISDNAGGIAEMAGMSHRIRERTDALDAA
GNTTAAIGKGFAIGSAALVSLALFGAFVSRAGIHTVDVLTPKVIIGLLVGAMLPYWFSAMTMKSVGS
AALKMVEEVRRQFNTIPGLMEGTAKPDYATCVKISTDASIKEMIPPGCLVMLTPLIVGFFFGVETLS
GVLAGSLVSGVQIAISASNTGGAWDNAKKYIEAGVSEHAKSLGPKGSEPHKAAVIGDTIGDPLKDTS
GPSLNILIKLMAVESLVFAPFFATHGGILFKYF

### SNAC1 - Oryza sativa (japonica cultivar-group) stress-induced Transcription factor NAC1 (SNAC1)

SEQ ID NO: 19: Complete CDS, codon optimized for *Nicotiana benthamiana,* Synthesized by Entelechon
   GenBank: DQ394702.1 
ATGGGAATGAGACGAGAACGAGACGCAGAAGCCGAGCTTAATCTGCCACCTGGCTTTCGTTTTCATC
CGACTGATGATGAACTAGTGGAACACTACCTTTGCAGAAAAGCAGCTGGTCAAAGGCTCCCAGTTCC
TATCATAGCTGAGGTTGATCTCTATAAATTTGACCCCTGGGATCTTCCGGAAAGGGCATTGTTTGGG
GCTCGTGAATGGTACTTTTTCACCCCACGCGATAGGAAGTACCCAAACGGATCTCGTCCAAACAGGG
CCGCTGGAAACGGTTATTGGAAAGCGACTGGAGCTGACAAGCCAGTAGCTCCTAGGGGGAGAACGCT
CGGTATTAAAAAGGCTCTAGTTTTCTACGCTGGAAAAGCCCCAAGAGGCGTTAAGACTGACTGGATT
ATGCATGAATATAGACTGGCTGATGCTGGAAGAGCTGCTGCGGGGGCTAAGAAAGGTAGTTTGAGAT
TAGACGATTGGGTCCTCTGTCGGCTATATAACAAGAAGAACGAGTGGGAGAAGATGCAACAGGGGAA
AGAGGTTAAGGAGGAAGCCTCGGATATGGTGACTTCCCAGTCGCATAGCCACACCCACTCTTGGGGA
GAAACCCGCACACCAGAGTCTGAGATCGTTGATAACGACCCCTTTCCGGAATTGGATTCTTTCCCTG
CCTTTCAACCTGCACCTCCACCTGCAACTGCAATGATGGTCCCGAAGAAAGAATCAATGGACGACGC
AACAGCAGCAGCTGCTGCTGCCGCGACAATCCCTAGGAATAATTCAAGCCTTTTCGTAGATCTTTCC
TACGACGATATTCAGGGGATGTATAGCGGCTTAGACATGTTGCCTCCCGGCGACGACTTCTATTCCT
CATTGTTCGCAAGTCCCCGTGTCAAAGGAACAACACCTCGAGCAGGTGCTGGAATGGGAATGGTGCC
TTTTTAA
SEQ ID NO: 20: Amino acid sequence

MGMRRERDAEAELNLPPGFRFHPTDDELVEHYLCRKAAGQRLPVPIIAEVDLYKFDPWDL
PERALFGAREWYFFTPRDRKYPNGSRPNRAAGNGYWKATGADKPVAPRGRTLGIKKALVF
YAGKAPRGVKTDWIMHEYRLADAGRAAAGAKKGSLRLDDWVLCRLYNKKNEWEKMQQGKE
VKEEASDMVTSQSHSHTHSWGETRTPESEIVDNDPFPELDSFPAFQPAPPPATAMMVPKK
ESMDDATAAAAAAATIPRNNSSLFVDLSYDDIQGMYSGLDMLPPGDDFYSSLFASPRVKG
TTPRAGAGMGMVPF-

### IPT isopentenyl transferase IPT (E88G) from Agrobacterium tumefaciens C58

SEQ ID NO: 21: CDS, E88G Mutation Gen Bank: AB025109.1 
Atggatctgcgtctaatttcggtccaacttgcacaggaaagacgtcgaccgcggtagctcttgccc
agcagactgggcttccagtcctttcgctcgatcgggtccaatgttgtcctcagctgtcaaccggaag
cggacgaccaacagtggaagaactgaaaggaacgagccgtctataccttgatgatcggcctctggtg
aagggtatcatcgcagccaagcaagctcatgaaaggctgatgggggaggtgtataattatgaggccc
acggcgggcttattcttgagggaggatctatctcgttgctcaagtgcatggcgcaaagcagttattg
gagtgcggattttcgttggcatattattcgccacgagttagcagacgaggagaccttcatgaacgtg
gccaaggccagagttaagcagatgttacgccctgctgcaggcctttctattatccaagagttggttg
atctttggaaagagcctcggctgaggcccatactgaaagagatcgatggatatcgatatgccatgtt
gtttgctagccagaaccagatcacatccgatatgctattgcagcttgacgcagatatggaggataag
ttgattcatgggatcgctcaggagtatctcatccatgcacgccgacaagaacagaaattccctcgag
ttaacgcagccgcttacgacggattcgaaggtcatccattcggaatgtat
SEQ ID NO: 22: Amino acid sequence

MDLRLIFGPTCTGKTSTAVALAQQTGLPVLSLDRVQCCPQLSTGSGRPTVEELKGTSRLYLDDRPLV
KGIIAAKQAHERLMGEVYNYEAHGGLILEGGSISLLKCMAQSSYWSADFRWHIIRHELAHEETFMNV
AKARVKQMLRPASGLSIIQELVDLWKEPRLRRILKEIDGYRYAMLFVSQNQITSDMLLQLDADMEDK
LIHGIAQEYLIHARRQEQKFPRVNAAAYDGFEGHPFGMY

### LeCYP - Tomato Cyclophilin

SEQ ID NO: 23: CDS
   M550019.1; gi: 170439 
Atggcaaatccaaaggtttctttgaccttaccatcggtggtgcaccagctggtcgtgtggtgatgg
agctcttcgccgataccactcccaaaaccgctgagaacttccgagctctttgtaccggtgagaaagg
tgttggaaagatggggaagcctttgcactacaaggctcaaccttccaccgtgtgatcccaggttc
atgtgtcaaggaggtgatttcaccgccggaaacggaccggaggagagtcgatctatggagccaaat
tcaacgatgagaacttcgttaagaagcacaccggccctggaatcctctccatggctaatgctggacc
tggaaccaacggttctcagtttttcatctgtaccgctaagactgagtggctcaacggaaagcacgtc
gtgtttggacaagttgttgaaggcatggatgtgattaagaaggcagaggctgttggatctagctctg
gaaggtgctccaagcctgtggttattgctgactgcggtcaactc
SEQ ID NO: 24: Amino acid sequence 
MANPKVFFDLTIGGAPAGRVVMELFADTTPKTAENFRALCTGEKGVGKMGKPLHYKGSTFHRVIPGF
MCQGGDFTAGNGTGGESIYGAKFNDENFVKKHTGPGILSMANAGPGTNGSQFFICTAKTEWLNGKHV
VFGQVVEGMDVIKKAEAVGSSSGRCSKPVVIADCGQL
SEQ ID NO: 25: T-DNA region of pNMD2492
SEQ ID NO: 26: T-DNA region of pNMD035
SEQ ID NO: 27: T-DNA region of pNMD661
SEQ ID NO: 28: T-DNA region of pNMD670
SEQ ID NO: 29: T-DNA region of pNMD694
SEQ ID NO: 30: T-DNA region of pNMD3486
SEQ ID NO: 31: T-DNA region of pNMD3493

## Claims

1. A process of providing a plurality of plants with improved drought stress tolerance, wherein the plants are monocot or dicot plants, comprising
(a) growing said plurality of plants on a farm field;
(b) determining whether drought stress affects said plants;
(c) providing aerial parts of said plants with a suspension containing cells of an *Agrobacterium* strain comprising a nucleic acid molecule comprising a nucleic acid construct containing a nucleotide sequence of interest, said nucleotide sequence of interest providing said plants with increased drought stress resistance upon expression in said plants, wherein said plants are transiently transfected with said nucleic acid molecule.

2. The process according to claim 1, wherein the presence or absence of drought stress affecting said plants is determined using one or more of the following criteria: water status in the soil such as soil moisture content, water status in tissue of the plants such as relative water content, a plant water stress index, turgor pressure, or stromatal conduct, and water potential in soil or the plant.

3. The process according to claim 1 or 2, wherein said nucleotide sequence of interest encodes a protein capable of providing said plant(s) with said increased drought stress tolerance;
wherein said protein preferably
i. has an amino acid sequence of any one of the amino acid sequences of SEQ ID NO: 2, 4, 6, 8, 10, 12, 14, 16, 18, 20, 22 and 24; or
ii. has an amino acid sequence having at least 80% sequence identity to the entire amino acid sequence of any one of SEQ ID NO: 2, 4, 6, 8, 10, 12, 14, 16, 18, 20, 22, and 24; or
iii. has an amino acid sequence of a length of at least 80 %, preferably at least 90 %, more preferably at least 95% of the number of amino acid residues of any one of SEQ ID NO: 2, 4, 6, 8, 10, 12, 14, 16, 18, 20, and 22, and a sequence identity of at least 90% to SEQ ID NO: 2, 4, 6, 8, 10, 12, 14, 16, 18, 20, 22, or 24 respectively, over such length; or
iv. has an amino acid sequence having from 1 to 20 amino acid additions, substitutions or deletions compared to the amino acid sequence of any one of SEQ ID NO: 2, 4, 6, 8, 10, 12, 14, 16, 18, 20, 22, and 24.

4. The process according to claim 3, wherein said protein is CspB from *B. subtilis.*

5. The process according to claim 3, wherein said protein is plant transcription factor LAS of SEQ ID NO: 8 or GmRE222 of SEQ ID NO: 10 or a variant thereof as defined in items ii to iv of claim 3.

6. The process according to any one of claims 1 to 5, wherein said plant(s) is (are) dicot, preferably Solanaceae, more preferably tomato plants.

7. The process according to any one of claims 1 to 6, wherein said nucleic acid construct is present in T-DNA and flanked on both sides by T-DNA border sequences, said T-DNA not containing a selectable marker allowing selection of plant or plant cell containing said T-DNA.

8. The process according to any one of claims 1 to 7, wherein said nucleotide sequence of interest is operably linked to a promoter active in plant cells.

## Patentansprüche

1. Verfahren zur Bereitstellung einer Vielzahl von Pflanzen mit verbesserter Trockenstress-Toleranz, wobei die Pflanzen einkeimblättrige oder zweikeimblättrige Pflanzen sind, umfassend:
(a) Wachsenlassen der Vielzahl der Pflanzen auf einem Feld eines Landwirtschaftsbetriebs;
(b) Bestimmen, ob Trockenstress die Pflanzen beeinträchtigt;
(c) Versehen der oberirdischen Teile der Pflanzen mit einer Suspension, die Zellen eines *Agrobacterium-Stamms* enthält, der ein Nukleinsäuremolekül umfasst, welches ein Nukleinsäurekonstrukt umfasst, das eine gewünschte Nukleotidsequenz enthält, wobei die gewünschte Nukleotidsequenz die Pflanzen mit einer erhöhten Trockenstress-Resistenz nach der Expression in den Pflanzen versieht, wobei die Pflanzen transient mit dem Nukleinsäuremolekül transfiziert werden.

2. Verfahren nach Anspruch 1, wobei das Vorliegen oder Nicht-Vorliegen von Trockenstress, der die Pflanzen beeinträchtig, unter Verwenden eines oder mehrerer der folgenden Kriterien bestimmt wird: Wasserstatus im Boden, wie der Feuchtigkeitsgehalt des Bodens, Wasserstatus im Gewebe der Pflanzen, wie der relative Wassergehalt, Wasserstress-Index der Pflanze, Turgor-Druck oder Leitung des Stromas und das Wasserpotenzial im Boden oder der Pflanze.

3. Verfahren nach Anspruch 1 oder 2, wobei die gewünschte Nukleotidsequenz für ein Protein codiert, das imstande ist die Pflanze(n) mit der verbesserten Trockenstress-Toleranz zu versehen, wobei das Protein bevorzugt
i. eine Aminosäuresequenz einer beliebigen der Aminosäuresequenzen der SEQ ID Nr. 2, 4, 6, 8, 10, 12, 14, 16, 18, 20, 22 und 24 aufweist oder
ii. eine Aminosäuresequenz mit wenigstens 80 % Sequenzidentität zu der gesamten Aminosäuresequenz einer beliebigen der SEQ ID Nr. 2, 4, 6, 8, 10, 12, 14, 16, 18, 20, 22 und 24 aufweist oder
iii. eine Aminosäuresequenz einer Länge von wenigstens 80 %, bevorzugt wenigstens 90 %, stärker bevorzugt wenigstens 95 % der Anzahl der Aminosäurereste einer beliebigen der SEQ ID Nr. 2, 4, 6, 8, 10, 12, 14, 16, 18, 20 und 22 aufweist und eine Sequenzidentität von wenigstens 90 % zu der SEQ ID Nr. 2, 4, 6, 8, 10, 12, 14, 16, 18, 20, 22 beziehungsweise 24 über eine solche Länge aufweist oder
iv. eine Aminosäuresequenz aufweist, die von 1 bis 20 Aminosäure-Insertionen, - Substitutionen oder -Deletionen im Vergleich zu der Aminosäuresequenz einer beliebigen der SEQ ID Nr. 2, 4, 6, 8, 10, 12, 14, 16, 18, 20, 22 und 24 aufweist.

4. Verfahren nach Anspruch 3, wobei das Protein CspB von *B. subtilis* ist.

5. Verfahren nach Anspruch 3, wobei das Protein der pflanzliche Transkriptionsfaktor LAS der SEQ. ID Nr. 8 oder GmRE222 der SEQ ID Nr. 10 oder eine Variante derselben, wie in den Punkten ii. bis iv. des Anspruchs 3 definiert, ist.

6. Verfahren nach einem der Ansprüche 1 bis 5, wobei die Pflanze eine zweikeimblättrige Pflanze ist (zweikeimblättrige Pflanzen sind), bevorzugt Solanaceae, stärker bevorzugt Tomatenpflanzen.

7. Verfahren nach einem der Ansprüche 1 bis 6, wobei das Nukleinsäurekonstrukt in der T-DNA vorliegt und auf beiden Seiten von T-DNA-Grenzsequenzen flankiert wird, wobei die T-DNA keine selektierbaren Marker enthält, die die Selektion von Pflanzen oder Pflanzenzellen ermöglicht, die die T-DNA enthalten.

8. Verfahren nach einem der Ansprüche 1 bis 7, wobei die gewünschte Nukleotidsequenz operativ mit einem Promotor verbunden ist, der in Pflanzenzellen aktiv ist.

## Revendications

1. Procédé pour doter une pluralité de plantes d'une meilleure tolérance au stress dû à une sécheresse, dans lequel les plantes sont des plantes monocotylédones ou dicotylédones, consistant à
(a) faire pousser ladite pluralité de plantes dans un champ agricole ;
(b) déterminer si un stress dû à une sécheresse affecte lesdites plantes ;
(c) fournir à des parties aériennes desdites plantes une suspension contenant des cellules d'une souche d'*Agrobacterium* comprenant une molécule d'acide nucléique comprenant un produit d'assemblage d'acide nucléique contenant une séquence de nucléotides présentant un intérêt, ladite séquence de nucléotides présentant un intérêt dotant lesdites plantes d'une meilleure résistance au stress dû à une sécheresse suite à l'expression dans lesdites plantes, lesdites plantes étant transfectées de façon transitoire par ladite molécule d'acide nucléique.

2. Procédé selon la revendication 1, dans lequel la présence ou l'absence de stress dû à une sécheresse affectant lesdites plantes est déterminée par utilisation d'un ou plusieurs des critères suivants : le statut hydrique du sol, tel que la teneur en humidité du sol, le statut hydrique dans le tissu des plantes tel que la teneur relative en eau, l'indice de stress hydrique des plantes, la pression de turgescence, ou le comportement des stromas, et le potentiel hydrique dans le sol ou les plantes.

3. Procédé selon la revendication 1 ou 2, dans lequel ladite séquence de nucléotides présentant un intérêt code une protéine capable de doter à ladite ou auxdites plante(s) une meilleure tolérance au stress dû à une sécheresse ;
dans lequel ladite protéine de préférence
i. possède une séquence d'acides aminés de l'une quelconque des séquences d'acides aminés des SEQ ID NO : 2, 4, 6, 8, 10, 12, 14, 16, 18, 20, 22 et 24 ; ou
ii. possède une séquence d'acides aminés ayant une identité de séquence d'au moins 80 % avec la séquence d'acides aminés complète de l'une quelconque des SEQ ID NO : 2, 4, 6, 8, 10, 12, 14, 16, 18, 20, 22 et 24 ; ou
iii. possède une séquence d'acides aminés ayant une longueur d'au moins 80 %, de préférence d'au moins 90 %, plus préférablement d'au moins 95 % du nombre de résidus d'acides aminés de l'une quelconque des SEQ ID NO : 2, 4, 6, 8, 10, 12, 14, 16, 18, 20 et 22, et une identité de séquences d'au moins 90 % avec la SEQ ID NO : 2, 4, 6, 8, 10, 12, 14, 16, 18, 20, 22 ou 24 respectivement, sur cette longueur ; ou
iv. possède une séquence d'acides aminés ayant de 1 à 20 additions, substitutions ou délétions d'acides aminés en comparaison avec la séquence d'acides aminés de l'une quelconque des SEQ ID NO : 2, 4, 6, 8, 10, 12, 14, 16, 18, 20, 22 et 24.

4. Procédé selon la revendication 3, dans lequel ladite protéine est CspB de *B. subtilis.*

5. Procédé selon la revendication 3, dans lequel ladite protéine est le facteur de transcription de plante LAS de la SEQ ID NO : 8 ou GmRE222 de la SEQ ID NO : 10 ou un variant de celui-ci comme défini dans les points ii à iv de la revendication 3.

6. Procédé selon l'une quelconque des revendications 1 à 5, dans lequel ladite ou lesdites plante (s) est ou sont dicotylédones, de préférence Solanaceae, plus préférablement des plants de tomate.

7. Procédé selon l'une quelconque des revendications 1 à 6, dans lequel ledit produit d'assemblage d'acide nucléique est présent dans l'ADN-T et est flanqué sur les deux côtés par des séquences de bordure d'ADN-T, ledit ADN-T ne contenant pas un marqueur sélectionnable permettant la sélection d'une plante ou d'une cellule de plante contenant ledit ADN-T.

8. Procédé selon l'une quelconque des revendications 1 à 7, dans lequel ladite séquence de nucléotides présentant un intérêt est liée de manière fonctionnelle à un promoteur actif dans des cellules de plante.
